(19) Europäisches Patentamt
European Patent Office
Office européen des brevets

(11) EP 2 493 461 B1

(12) **EUROPEAN PATENT SPECIFICATION**

(45) Date of publication and mention of the grant of the patent:
**06.01.2016 Bulletin 2016/01**

(21) Application number: **10826225.4**

(22) Date of filing: **28.10.2010**

(51) Int Cl.:
***A61K 31/745*** (2006.01) ***A61K 31/01*** (2006.01)
***A61K 31/015*** (2006.01) ***A61P 17/02*** (2006.01)
***A61P 17/06*** (2006.01) ***A61P 17/10*** (2006.01)
***A61P 25/18*** (2006.01) ***A61P 25/28*** (2006.01)
***A61P 25/16*** (2006.01) ***A61K 31/765*** (2006.01)

(86) International application number:
**PCT/IL2010/000893**

(87) International publication number:
**WO 2011/051945 (05.05.2011 Gazette 2011/18)**

(54) **POLYMERIC MONOTERPENES FOR TREATING IMPAIRED NEUROLOGICAL FUNCTION**

POLYMERE MONOTERPENE ZUR BEHANDLUNG EINGESCHRÄNKTER NEUROLOGISCHER FUNKTION

MONOTERPÈNES POLYMÈRES POUR LE TRAITEMENT D'UNE FONCTION INHIBÉE NEUROLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.10.2009 US 272737 P**

(43) Date of publication of application:
**05.09.2012 Bulletin 2012/36**

(73) Proprietor: **Regenera Pharma Ltd.**
**76326 Rehovot (IL)**

(72) Inventor: **HAZAN, Zadik**
**30900 Zichron Yaakov (IL)**

(74) Representative: **Pohlman, Sandra M.**
**df-mp Dörries Frank-Molnia & Pohlman**
**Patentanwälte Rechtsanwälte PartG mbB**
**Theatinerstrasse 16**
**80333 München (DE)**

(56) References cited:
**WO-A2-2008/036932**
**WO-A2-2010/100650**
**WO-A2-2010/100651**
**US-A- 4 347 356**
**US-A- 5 288 488**
**US-A1- 2004 052 922**
**US-A1- 2006 222 723**
**US-A1- 2006 241 130**
**US-A1- 2007 009 463**
**US-A1- 2009 209 720**
**US-A1- 2009 246 327**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION uses

The invention relates to therapeutic uses of polymeric forms of the monoterpene compounds alloocimene, limonene, α-pinene, β-pinene, geranyl acetate, 3-carene and 2-carene i.e. to a composition comprising isolated polymeric forms of monoterpenes in a suitable carrier for use in treating impaired neurological function.

### BACKGROUND OF THE INVENTION

Various monoterpenes have been isolated from plant sources and/or chemically synthesized. Uses of monoterpenes in food products, perfumery, hygienic products and agrochemicals have been described.

U.S. Patent No. 7,780,974 discloses saponin compounds comprising a triterpene moiety, which is typically an acacic or oleanolic acid, and further comprising a monoterpene moiety, such as trans-2-hydroxymethyl-6-methyl-6-hydroxy-2,7-octadienoic or trans-2,6-dimethyl-6-hydroxy-2,7-octadienoic acid. Further disclosed is the use of the compounds for coating stents, use thereof for therapy of restenosis, and the activity of the compounds as anti-inflammatory and anti-stress agents and for preventing abnormal proliferation of mammalian epithelial cells.

U.S. Patent No. 6,063,383 discloses a pharmaceutical suppository composite for treatment of fever and influenza which comprises volatile oil of radix bupleuri scorzonerifolium wild, wherein said volatile oil contains various terpene compounds such as β-terpinene, limonene, camphene, β-fenchene, pulegone, isoborneol, β-terpineol, linalool, α-copaene, humulene, α-farnesene, aromadendrene, cis-caryophyllene, iso-caryophyllene, among others.

U.S. Patent Application Publication No. 2009/0304799 discloses an influenza nanoemulsion vaccine comprising a volatile oil, inter alia a monoterpene.

U.S. Patent Application Publication No. 2006/0222723 discloses a composition of terpene-based substances derived from natural resins, such as olibanum resin, myrrh resin, and Dacryoides klaineana resin. According to the disclosure, a sesquiterpene fraction of U.S. Patent Application Publication No. 2004/052922 relates to pharmaceutical and dietary compositions, as well as to functional foodstuffs useful as coadjuvants for both treating and preventing aging processes and related conditions: atherosclerosis, hypertension, diabetes, tumors, obesity and overweight, hypertriglyceridemia, hypercholesterolemia, aging of the skin, alopecia, panniculopathia (cellulite), osteoporosis, cerebral aging (Alzheimer, Parkinson, senile dementia, etc.) and loss of memory, stress, depression, menopausal syndromes, benign prostate hypertrophy, etc.. Said compositions comprise: a) a lipidic mixture rich in polyunsaturated acids and vitamins, in combination with at least two components selected from; b) one or more terpenes selected from monoterpenes and/or sesquiterpenes; c) 1-piperoylpiperidine in the pure form and/or extracts or purified fractions enriched in black pepper containing it; or d) one or more polycosanols and/or polycosanolic acids. Dacryoides may contain oligomers or polymers of sesquiterpenes and monoterpenes. The composition is reportedly useful for the treatment of diseases of the central and peripheral nervous systems, inter alia anxiety, depression, epilepsy, schizophrenia, Parkinson's disease, multiple sclerosis, Alzheimer disease, lateral amyotrophic sclerosis, drug dependency and brain tumor.

U.S. Patent Application Publication No. 2006/0104997 discloses a pharmaceutical composition comprising: a monoterpene or a derivative thereof, one or more surfactants, and optionally one or more cosolvents, and use thereof for treating neoplastic disease. According to the disclosure, the monoterpene may be perillyl alcohol (1-hydroxymethyl-4-isopropenyl-1-cyclohexene), (R)-1-methyl-4-(1-methylethenyl)cyclohexene (d-limonene), 1-methyl-4-hydroxypropyl-1-cyclohexene (α-terpineol), carveol, carvone, dihydrocarveol, dihydrocarvone, pulegone, isopulegol, menthol, menthone, terpinen-4-ol, sobrerol, limonene oxide, uroterpenol, perillaldehyde, dihydroperillic acid, dihydroperillic acid methyl ester, β-myrcene, perillic acid-8,9-OH, α-pinene, linalool or perillic acid, among others.

WO 2004/066912 discloses a method of treating a medical condition associated with inflammation, comprising administering a compound inter alia a monoterpene such as citronellol, geraniol, nerol, linalool, citral, carvone, pulegone, limonene, myrcene, α-terpinene, γ-terpinene, terpinolene, careen, terpinol, α-terpinol, α-thujene, α-pinene and β-pinene, among others. According to the disclosure, the disease may be an inflammatory neurological disease, inter alia multiple sclerosis, Alzheimer's disease, Parkinson's disease, myasthenia gravis, motor neuropathy and Guillain-Barre syndrome.

WO 2002/051395 discloses a method for increasing the differentiation of mammalian neuronal cells and for alleviating a neurodegenerative disease, inter alia Parkinson's disease, amyotrophic lateral sclerosis or Alzheimer's disease, comprising use of a large number of C3-C50 diol compounds, inter alia (1R)-2-pinene-10-ol.

WO 2008/070783 discloses a composition comprising a gingerol, and further comprising an essential oil inter alia phellandrene, limonene or β-pinene. Further disclosed is use of the composition for treatment of Alzheimer's disease and Parkinson's disease and for skin protection.

WO 1998/000168 discloses a composition comprising a topically applicable antihistaminic compound in combination with a terpenoid compound, inter alia 3-carene or limonene for the topical treatment of allergic and inflammatory skin diseases.

U.S. Patent Application Publication No. 2008/0121139 discloses a coating comprising a polymer comprising a terpene and a monomer that is polymerized with terpene by free radical polymerization, wherein the terpene may comprise α-pinene, β-pinene or limonene.

U.S. Patent No. 5,776,361 discloses an oxygen scavenging composition comprising at least one polyterpene, inter alia poly(α-pinene), poly(dipentene), poly(β-pinene), poly(d-limonene) or poly(d,1-limonene); and at least one catalyst for use as a coating on aluminum foil or paper, or formed into bottles or other rigid containers.

U.S. Patent No. 5,154,927 discloses a chewing gum with a controlled release active ingredient wherein the chewing gum base comprises polymeric beads comprising a copolymer of monomer pairs selected from the group consisting of styrene and divinylbenzene, limonene and divinylbenzene, carvone and divinylbenzene, eugenol and divinylbenzene, and ocimene and divinylbenzene.

The effect of alloocimene on healing of skin and skin-muscle wounds has been described (Pravdich-Neminskaya et al., Bulletin of Experimental Biology and Medicine Volume 85, Number 1, 57-60, January 1978).

U.S. Patent No. 3,979,371 discloses a limonene epoxide polymer and preparation thereof, useful as a tackifier in an adhesive composition for rubbers or elastomers.

U.S. Patent No. 2,264,774 discloses a process for polymerizing a terpene, such as β-pinene.

U.S. Patent No. 4,165,301 discloses a compounded single phase liquid perfumery composition comprising inter alia dimerization products of various terpenes, such as of α-pinene, β-pinene, camphene, d-limonene, or of turpentine.

U.S. Patent No. 6,265,478 discloses a polymeric resinous material comprising units derived from limonene, and use thereof in a pneumatic tire.

Liquid-crystalline polymers with a backbone of limonene-co-methyl methacrylate have been disclosed (Mishra et al., J Appl Polym Sci 102: 4595-4600,2006).

The synthesis of polymers of β-pinene and α-phellandrene via cationic polymerization has been disclosed (Green Chem. 2006, vol. 8, pp.878-882).

U.S. Patent Application Publication No. 2009/0209720 discloses a pinene polymer having a weight-average molecular weight of 90,000 to 1,000,000, obtained by polymerizing β-pinene in the presence of a bifunctional vinyl compound.

U.S. Patent No. 4,694,047 halogenated poly(alloocimene) and use thereof in coatings and slow release material for pesticides.

The prior art does not contain any teaching or suggestion of the therapeutic uses for oligomeric or polymeric monoterpenes whether those derived from plants or obtained by chemical synthesis, as active ingredients in a pharmaceutical composition or in a therapeutic application for treating neurological metabolic conditions

## SUMMARY OF THE INVENTION

The scope of the present invention is defined by the appended claims.

The present invention is based in part on the unexpected discovery that polymeric forms of the cyclic and acyclic monoterpene compounds alloocimene, limonene, α-pinene (also denoted herein as alpha-pinene), β-pinene (also denoted herein as beta-pinene), geranyl acetate, 3-carene and 2-carene, exhibit beneficial biological activities which may be exploited for a variety of therapeutic applications. More specifically, synthetically produced polymeric forms of each of these monoterpenes were found to have potent activity in inducing regeneration or differentiation of a variety of cell types, including neuronal, endothelial and epidermal cells, including those of ectodermal, mesodermal and endodermal lineages.

It is thus disclosed for the first time that polymeric forms of these specific monoterpenes can be employed as an active ingredient in pharmaceutical compositions for treating neurodegenerative disorders such as Alzheimer's disease, as well as for inducing tissue regeneration, for example for treating skin disorders including chronic wounds. Moreover, the inventors of the present invention have shown that such polymers having degrees of polymerization in the range from about 6 to about 200, exhibit superior activity over the corresponding monoterpenes in monomeric form, the latter of which failed to exhibit the activities ascribed to the subject compounds disclosed herein.

The teachings of the present invention have been exemplified with synthetic polymeric monoterpenes synthesized from limonene, pinene, alloocimene and geranyl acetate monomers. Moreover, the teachings of the present invention are particularly surprising and unexpected over the prior art, the latter of which teaches some therapeutic uses of monoterpene monomers, but not of polymers .

Without wishing to be bound by any particular theory or mechanism of action, the activity of polymeric monoterpenes in inducing neuronal cell differentiation, as disclosed herein, renders the present invention useful for reformation of inter-neuronal junctions and overcoming defective inter-neuronal communication in brain and neural tissue affected by pathologies associated with inadequate synaptic formation. This pathology underlies many nervous system pathologies, including for example Alzheimer's disease and stroke, which can benefit from the regenerative and trophic effects

of the compounds.

Further, the invention may be used for reversing adverse effects of various drugs which act on the nervous system, such as anesthetics. The provided polymers are further useful for promoting wound healing and rejuvenation of a large number of cells and tissues.

As used herein "polymeric monoterpenes" encompass polymeric forms of monoterpenes having a degree of polymerization of at least 6. Polymeric monoterpenes include those formed from alloocimene, limonene, α-pinene, β-pinene, geranyl acetate, 3-carene and 2-carene, and further encompass homopolymers thereof as well as heteropolymers thereof.

It is to be understood explicitly that the use of polymeric forms of myrcene is not encompassed within the scope of the present invention.

According to a first aspect, the present invention provides a composition comprising at least one polymeric form of a monoterpene, for use in the treatment of impaired neurological function wherein the monoterpene is selected from the group consisting of alloocimene, limonene, α-pinene, β-pinene, geranyl acetate, 3-carene and 2-carene; and a pharmaceutically acceptable carrier. Each possibility is a separate embodiment of the invention.

Furthermore, herein described is a composition comprising at least one polymeric form of a monoterpene, for use in the treatment of a skin or scalp disorder wherein the monoterpene is selected from the group consisting of alloocimene, limonene, α-pinene, β-pinene, geranyl acetate, 3-carene and 2-carene; and a pharmaceutically acceptable carriers.

According to yet another aspect the invention provides a composition comprising at least one polymeric form of a monoterpene for use in inducing a regenerative process, wherein the monoterpene is selected from the group consisting of alloocimene, limonene, α-pinene, β-pinene, geranyl acetate, 3-carene and 2-carene; and a pharmaceutically acceptable carrier. Each possibility is a separate embodiment of the invention.

In particular embodiments, the composition comprises a polymeric monoterpene selected from the group consisting of polymeric alloocimene, polymeric limonene, polymeric α-pinene, polymeric β-pinene, polymeric geranyl acetate, polymeric 3-carene, polymeric 2-carene and combinations thereof. Each possibility is a separate embodiment of the invention.

In particular embodiments, the polymeric monoterpene is selected from polymeric alloocimene and polymeric limonene.

In particular embodiments, the composition is substantially devoid of the corresponding monomeric form of the monoterpene.

In particular embodiments, the composition comprises said polymeric monoterpene as the sole active ingredient.

In particular embodiments, the composition comprises polymeric alloocimene as the sole active ingredient. In particular embodiments, the composition comprises polymeric limonene as the sole active ingredient.

In particular embodiments, the composition consists of a polymeric monoterpene selected from the group consisting of polymeric alloocimene, polymeric limonene, polymeric α-pinene, polymeric P-pinene, polymeric geranyl acetate, polymeric 3-carene, polymeric 2-carene and combinations thereof; and a pharmaceutically acceptable carrier. Each possibility is a separate embodiment of the invention. In particular embodiments, the polymeric monoterpene is selected from polymeric alloocimene and polymeric limonene.

In particular embodiments, the composition consists of polymeric alloocimene and a pharmaceutically acceptable carrier. In particular embodiments, the composition consists of polymeric limonene and a pharmaceutically acceptable carrier.

In particular embodiments, the composition is substantially devoid of a gingerol. In particular embodiments, the composition is substantially devoid of a monoterpene in monomeric form, wherein said monoterpene is selected from the group consisting of alloocimene, limonene, α-pinene, β-pinene, geranyl acetate, α-phellandrene, γ-terpinene, 3-carene, 2-carene and combinations thereof. Each possibility is a separate embodiment of the invention.

In particular embodiments, a use comprising administering a therapeutically effective amount of a composition consisting of at least one of: polymeric alloocimene, polymeric limonene, polymeric α-pinene, polymeric β-pinene, polymeric geranyl acetate, polymeric 3-carene, or polymeric 2-carene; and a pharmaceutically acceptable carrier; wherein the composition is substantially devoid of the corresponding monomeric form of said monoterpene.

In particular embodiments, the composition comprises less than 1% (w/w) of the corresponding monomeric form of said monoterpene.

In particular embodiments, the composition comprises less than 0.5% (w/w) of the corresponding monomeric form of said monoterpene.

In particular embodiments, the composition comprises less than 0.2% (w/w) of the corresponding monomeric form of said monoterpene.

In particular embodiment, the polymeric monoterpene is present in the composition in an amount from about 0.01 to about 12% (w/w), based on the total weight of the composition.

In a particular embodiment, the polymeric monoterpene has a degree of polymerization in the range of at least

about 6 to about 200. In a particular embodiment, the degree ,of polymerization is in the range from about 6 to about 50. In a particular embodiment, the degree of polymerization is at least about 25. In a particular embodiment, the degree of polymerization is in the range of about 30 to about 100, or in the range of about 50 to about 150.

In a particular embodiment, the polymeric monoterpene has a number average molecular weight of at least about 1000. In a particular embodiment, the polymeric monoterpene has a number average molecular weight of up to about 25,000.

In a particular embodiment, the number average molecular weight is at least about 3000. In a particular embodiment, the number average molecular weight is at least about 5000. In a particular embodiment, the polymeric monoterpene has a number average molecular weight in the range from at least about 1000 to about 25,000. In particular embodiments, the number average molecular weight is in a range selected from the group consisting of: at least about 1000 to about 5000; at least about 1000 to about 15,000; about 5000 to about 15,000; about 5000 to about 20,000; about 15,000 to about 25,000; and combinations thereof. Each possibility is a separate embodiment of the invention.

In a particular embodiment, the polymeric monoterpene has a molecular distribution less than 5.

In a particular embodiment, the polymeric monoterpene is a product of a chemical synthesis. In a particular embodiment, the polymeric form of said monoterpene is isolated from a botanical fraction. In a particular embodiment, the polymeric monoterpene is present in an isolated botanical fraction.

In another embodiment, the polymeric monoterpene is a product of a chemical synthesis and has a number average molecular weight in the range from about 1000 to about 25,000. In particular embodiments, the number average molecular weight is in a range selected from the group consisting of: at least about 1000 to about 5000; at least about 1000 to about 15,000; about 5000 to about 15,000; about 5000 to about 20,000; about 15,000 to about 25,000; and combinations thereof. Each possibility is a separate embodiment of the invention.

In a particular embodiment, the chemical synthesis comprises the use of a monomeric monoterpene as a substrate, wherein the monomeric monoterpene is selected from the group consisting of alloocimene, limonene, α-pinene, β-pinene, geranyl acetate, 3-carene and 2-carene. In a particular embodiment, the monomeric monoterpene substrate is derived from a plant species.

In a particular embodiment, the polymeric monoterpene is a product of a chemical synthesis and is substantially devoid of monomeric forms of the same monoterpene. In a particular embodiment, the polymeric monoterpene is a product of a chemical synthesis and the composition is substantially devoid of monomeric and oligomeric forms of the same monoterpene substrate.

In a particular embodiment, the chemical synthesis is selected from the group consisting of an anionic polymerization reaction, a cationic polymerization reaction, a radical polymerization, a metal-catalyzed polymerization, a transition metal catalyzed polymerization and a photopolymerization reaction.

In a particular embodiment, the pharmaceutically acceptable carrier comprises at least one oil. In a particular embodiment, the at least one oil is selected from the group consisting of a mineral oil, a vegetable oil and combinations thereof. In a particular embodiment, the vegetable oil is selected from the group consisting of almond oil, canola oil, coconut oil, corn oil, cottonseed oil, grape seed oil, olive oil peanut oil, saffron oil, sesame oil, soybean oil, and combinations thereof. In a particular embodiment, the mineral oil is light mineral oil.

In particular embodiments, the pharmaceutical composition is in a form selected from the group consisting of a capsule, a tablet, a suppository, a suspension and an ointment. In particular embodiments, the pharmaceutical composition comprises at least one of a liposome, a film, an emulsion, a microemulsion, a microcapsule and a cement.

In various embodiments, the step of administering is carried out by a route selected from the group consisting of topical, intramuscular, intravenous, intraperitoneal, subcutaneous, intradermal, vaginal, rectal, intracranial, intranasal, intraocular, and auricular. Each possibility is a separate embodiment of the invention.

In various embodiments, the step of administering is carried out by the oral route. In various embodiments, the step of administering is carried out by a route which is other than an oral or enteral route.

In particular embodiments, the step of administering comprises contacting cells with the composition, wherein the cells are of a particular type, of a particular lineage or at a particular stage of differentiation.

In particular embodiments, the cells are selected from the group consisting of neural cells, neuronal cells, endothelial cells, epithelial cells, osteoblasts and chondrocytes. In particular embodiments, the cells are of a lineage selected from the group consisting of ectodermal, mesodermal and entodermal lineages. In various embodiments, the step of contacting cells is carried out *in vivo, ex vivo* or *in vitro.*

In a particular embodiment, the cells are contacted *ex vivo* or *in vitro* with the composition, and are thereafter implanted or transplanted into the subject. In a particular embodiment, the cells for implantation or transplantation are of an organ or tissue. In a particular embodiment, the cells are those which secrete soluble factors.

In particular embodiments, the step of administering comprises withdrawing cells or body fluids from the subject or animal, contacting the cells or body fluids with the composition, and returning said cells or body fluids to the subject or animal.

In a particular embodiment, the impaired neurological function comprises a decrease in a function selected

from the group consisting of cognitive function, sensory function, motor function and combinations thereof. In particular embodiments, the impaired neurological function is associated with a condition or disease selected from the group consisting of vascular dementia, senile dementia, Alzheimer's disease, amyotrophic lateral sclerosis (ALS), Huntington's disease, multiple sclerosis and Parkinson's disease. Each possibility is a separate embodiment of the invention.

In a particular embodiment, the impaired neurological function is due to exposure to a drug, such as an anesthetic drug.

Skin and scalp disclosers include disorders of skin, scalp and hair appendages, including for example, nails and hair follicles. The skin or scalp disorder can be selected from the group consisting of alopecia, eczema, psoriasis, acne, seborrheic keratosis and seborrhea. The skin disorder can be a skin wound, including for example, a venous leg ulcer, a pressure ulcer, a diabetic foot ulcer, a burn, an amputation wound, a decubitus ulcer (bed sore), a split-skin donor graft, a skin graft donor site, a medical device implantation site, a bite wound, a frostbite wound, a puncture wound, a shrapnel wound, a dermabrasion, an infection wound and a surgical wound. In a particular embodiment, the source of the wound is selected from the group consisting of an infection; exposure to ionizing radiation; exposure to laser, and exposure to a chemical agent.

The uses disclosed herein, the step of administering or contacting cells may comprise the use of an article of manufacture, wherein the composition is disposed on or within the article of manufacture. In a particular embodiment, the composition is disposed on the article of manufacture in the form of a coating. In a particular embodiment, the article of manufacture comprises a vessel, wherein the composition is disposed within the vessel. In a particular embodiment, the article of manufacture is selected from the group consisting of a fabric article, a diaper, a wound dressing, a medical device, a needle or plurality of needles, a microneedle or plurality of microneedles, an injection device and a spray dispenser. In a particular embodiment, the article of manufacture comprises a plurality of microneedles.

In particular embodiments, the medical device is selected from the group consisting of a prosthetic, an artificial organ or component thereof, a valve, a catheter, a tube, a stent, an artificial membrane, a pacemaker, a sensor, an endoscope, an imaging device, a pump, a wire and an implant. In a particular embodiment, the implant is selected from the group consisting of a cardiac implant, a cochlear implant, a corneal implant, a cranial implant, a dental implant, a maxillofacial implant, an organ implant, an orthopedic implant, a vascular implant, an intraarticular implant and a breast implant.

In a particular embodiment, the composition is administered/contacted prior to or following implantation of a medical device into the subject. In a particular embodiment, the medical device is an organ implant. In a particular embodiment, the organ implant comprises autologous cells of the subject. In a particular embodiment, the method is carried out prior to or following transplantation of cells, tissue or an organ into the subject.

In a particular embodiment, the step of administering or contacting comprises a means selected from the group consisting of electroporation, sonication, radio frequency, pressurized spray and combinations thereof.

In a particular embodiment, the step of contacting comprises establishing contact between interstitial fluid and the composition. In a particular embodiment, the step of establishing contact between interstitial fluid and the composition comprises piercing and/or teasing the dermis with a needle, a microneedle, or an apparatus comprising a plurality of needles or microneedles.

In a particular embodiment, the subject is a human. In a particular embodiment, the subject is selected from a non-human mammal, a fish and a bird.

According to another aspect, the present invention provides use of a polymeric form of a monoterpene, wherein the monoterpene is selected from the group consisting of alloocimene, limonene, α-pinene, β-pinene, geranyl acetate, 3-carene and 2-carene, for the preparation of a medicament for treating impaired neurological function.

Further described is the use of polymeric form of a monoterpene, wherein the monoterpene is selected from the group consisting of alloocimene, limonene, α-pinene, β-pinene, geranyl acetate, 3-carene and 2-carene, for the preparation of a medicament for treating a skin or scalp disorder.

Claimed or described herein are also shorter and longer forms of polymeric monoterpenes of the monoterpenes described, including synthetic and semi-synthetic forms, including copolymers, and derivatives substituted with various functionalities, and conjugates with additional molecules, as are known in the art, with the stipulation that these variants and modifications preserve the therapeutic capacity of the polymeric material compounds in the context of the methods of the present invention.

Other objects, features and advantages of the present invention will become clear from the following description and drawings.

## BRIEF DESCRIPTION OF THE DRAWINGS

**Figure 1** shows monomeric forms of cyclic and acyclic monoterpenes which may be used to produce oligomers

and polymers.

**Figure 2** shows neuronal differentiation in ARPE-19 RPE cells induced by polymeric limonene. Fig. 2A shows cells that were treated with polymeric limonene. Fig. 2B shows cells that were treated with vehicle (cottonseed oil).

**Figure 3** shows neuronal differentiation in ARPE-19 RPE cells induced by polymeric alloocimene. Fig. 3A shows cells that were treated with polymeric alloocimene. Fig. 3B shows cells that were treated with vehicle (cottonseed oil).

**Figure 4** shows a comparison of the effects of polymeric alloocimene and monomeric alloocimene on ARPE-19 RPE cells. Cells were treated with polymeric alloocimene (Fig. 4A), monomeric alloocimene (Fig. 4B), or cottonseed oil vehicle (Fig. 4C).

**Figure 5** is a size exclusion chromatogram of chemically synthesized polymeric alloocimene, showing peaks at retention time (RT) 7.841 and 7.917 min.

**Figure 6** is a size exclusion chromatogram of chemically synthesized polymeric limonene, showing peaks at RT 8.493, 8.703 and 9.271 min.

## DETAILED DESCRIPTION OF THE INVENTION

The inventor of the present invention has surprisingly found that chemically synthesized polymeric forms of specific monoterpenes of various molecular weight ranges, have activity in ameliorating impaired neurological function, promoting neural cell differentiation; wound healing and ameliorating various skin conditions.

In particular, it is herein disclosed for the first time that owing to their various activities in stimulating and inducing cell regeneration, polymeric monoterpenes previously known only for use in industrial applications may be employed for therapeutic use in humans. Surprisingly, the polymeric compounds of the invention are advantageous over the corresponding monomeric monoterpenes, since the latter do not exhibit the cell stimulating activities disclosed herein.

Advantageously, the compositions may be used in treating impaired neurological function and skin and scalp conditions. Upon contact with cells of both human and non-human subjects, the composition induces cell differentiation in a wide array of tissues, cell compartments and cell lineages, including skin, endothelium, mucous membranes, bones, tendons and cartilage. In addition, the cell differentiation activity of the pharmaceutical composition may be exploited for promoting *in vivo* incorporation of medical devices, implants and organ transplants.

It is thus disclosed herein for the first time that polymeric forms of alloocimene, limonene, α-pinene, β-pinene, geranyl acetate, α-phellandrene, γ-terpinene, 3-carene and 2-carene, of various molecular weight ranges have activity in inducing differentiation in retinal pigmented epithelium cells. It has been also found that cell lines of melanoma and neuroblastoma were induced to differentiate, therefore reducing their malignant potential. For example, malignant melanoma cells treated with polymeric alloocimene assumed a less malignant morphology, and observed change in the differentiation was accompanied by typical melanin production in the cytoplasm.

### Definitions

As used herein, "terpene compounds" refers to isoprene-containing hydrocarbons, having isoprene units ($CH_2=C(CH_3)-CH=CH_2$) in a head-to-tail orientation. Terpene hydrocarbons in general, have the molecular formula $(C_5H_8)_n$, and include hemiterpenes, (C5), monoterpenes (C10), sesquiterpenes (C15), diterpenes (C20), triterpenes (C30), and tetraterpenes (C40) which respectively have 1, 2, 3, 4, 6 and 8 isoprene units. Terpenes may be further classified as acyclic or cyclic.

Examples of monoterpenes include geranyl acetate, alloocimene, limonene and pinene.

As used herein "polymeric monoterpenes" encompass polymeric forms of monoterpenes having a degree of polymerization of at least 6. Polymeric monoterpenes for use in the invention include those formed from alloocimene, limonene, polymeric α-pinene, β-pinene, geranyl acetate, 3-carene and 2-carene, either each on its own or in various combinations. Accordingly, polymeric monoterpenes encompass both homopolymers and heteropolymers (also known as copolymers). Also included are geometric isomers, optical isomers and diastereoisomers of these polymeric compounds.

The monoterpene polymer can be derived from a plant source or is a product of chemical synthetic reaction. The monoterpene polymer preferably has a defined molecular weight or molecular weight range.

As used herein, "homopolymer" refers to a polymer that is produced from a single type of monomer. For example, polymeric limonene is a homopolymer when it is produced only from limonene monomers, for example R+ limonene. A homopolymer may also be a mixture of polymers produced from the same monomer, but having a varying degree of

polymerization i.e. chain length. Accordingly, polymeric limonene for example may encompass a range of compounds of different chain lengths and accordingly different molecular weights. Further, a homopolymer may contain monomers having different isomeric configurations, for example, β- and α-isomers.

Homopolymers for use in the invention include polymeric alloocimene, polymeric limonene, polymeric α-pinenc, polymeric β-pinene, polymeric geranyl acetate, polymeric 3-carehe and polymeric 2-carene.

As used herein, "heteropolymer" and "copolymer" refer to a polymer produced from more than one type of monomer. Thus for example, a limonene copolymer is produced from limonene monomers, in addition to a heterologous type of monomer that is not limonene, for example pinene. Copolymers include alternating copolymers, periodic copolymers, random copolymers, block copolymers and statistical copolymers, as is known in the art.

As used herein, the term "oligomeric monoterpene" refers to oligomerized forms of monoterpenes having a degree of oligomerization in the range from 2 to 5. Oligomeric monoterpenes include those formed from alloocimene, limonene, α-pinene, β-pinene, geranyl acetate, α-phellandrene, γ-terpinene, 3-carene and 2-carene, either each on its own or in various combinations. Accordingly, oligomeric monoterpenes encompass both homo-oligomers and hetero-oligomers.

As used herein, "homo-oligomer" refers to an oligomer that is produced from units of a single type of monomer, for example only β-pinene.

As used herein, "heter-oligomer" refers to an oligomer that is produced from different types of monomer, for example from γ-terpinene and β-pinene.

As used herein, "degree of polymerization" refers to the number of monomers or monomeric units which are covalently associated together to form a polymer or an oligomer, for example, the number of limonene monomers in a polymeric limonene

As used herein, "weight average molecular weight" refers to the average molecular weight of a polymer having molecules of different chain lengths, as expressed by the equation:

$$\bar{M}_w = \frac{\sum_i N_i M_i^2}{\sum_i N_i M_i}$$

where $N_i$ is the number of molecules of molecular weight $M_i$. The weight average molecular weight can be determined for example, by light scattering, small angle neutron scattering, X-ray scattering, and sedimentation velocity.

As used herein, "number average molecular weight" refers to the average molecular weight of a polymer having molecules of different chain lengths, as expressed by the equation:

$$\bar{M}_n = \frac{\sum_i N_i M_i}{\sum_i N_i}$$

where $N_i$ is the number of molecules of molecular weight $M_i$. The number average molecular weight can be determined for example, by gel permeation chromatography (also known as size exclusion chromatography) or viscometry.

The terms "polydispersity index" and "molecular distribution" are herein used interchangeably to refer to the ratio of the weight average molecular weight to the number average molecular weight.

As used herein, "an oligomeric form" in reference to a monoterpene refers to an oligomeric monoterpene in which the monomeric units are either of the same monoterpene or of different monoterpenes, and are joined in any possible arrangements, and are connected one to another through any possible bond or functional group.

As used herein, "substantially devoid" means that a preparation or composition according to the invention contains less than 3% of the stated substance, preferable less than 1% and most preferably less than 0.5%.

As used herein, "substantially devoid of the corresponding monomeric form" means that a preparation or composition comprising an oligomeric or polymeric monoterpene according to the invention, contains less than 3% of the monomeric form of the same monoterpene constituting the subject oligomeric or polymeric form, preferably less than 1% and most preferably less than 0.5%.

As used herein, "therapeutically effective amount" refers to that amount of a pharmaceutical ingredient which substantially induces, promotes or results in a desired therapeutic effect.

As used herein, "pharmaceutically acceptable carrier" refers to a diluent or vehicle which is used to enhance the delivery and/or pharmacokinetic properties of a pharmaceutical ingredient with which it is formulated, but has no therapeutic effect of its own, nor does it induce or cause any undesirable or untoward effect or adverse reaction in the subject.

As used herein, "pharmaceutically acceptable hydrophobic carrier" refers to a hydrophobic non-polar diluent or vehicle in which the oligomeric or polymeric monoterpene of the invention is dissolved or suspended. In general, the pharmaceutically acceptable hydrophobic carrier does not interfere with the therapeutic activity of the aforementioned monoterpene, nor is it irritating or detrimental to the subject.

As used herein, "cell differentiation" refers to the process in which a less specialized cell becomes a more specialized cell. Cell differentiation may be established on the basis of changes in any of a number of cellular characteristics, including but not limited to size, shape, organelle appearance, membrane potential, metabolic activity, and responsiveness to signals. A particular "grade" may be given to a cell type to describe the extent of differentiation.

As used herein, "impaired neurological function" refers to a decline or decrease in at least one of sensory, cognitive or motor function, as compared to a previous level of function or activity, and/or as compared to non-impaired individuals matched according to accepted criteria.

Monoterpenes

Pinene ($C_{10}H_{16}$; mol mass 136.23) is a bicyclic monoterpene, of which the two structural isomers α-pinene (2,6,6,-trimethylbicyclo[3.2.1.] hept-2-ene) and β-pinene (6,6,dimethyl-2-methylenebicyclo[3.1.1.] heptane) exist in nature. Both forms are important constituents of resins of pine tree and many other conifers, and are also found in non-coniferous plant species.

Biosynthetically, α-pinene and β-pinene are both produced from geranyl pyrophosphate, via cyclisation of linaloyl pyrophosphate followed by loss of a proton from the carbocation equivalent.

Both forms of pinene are present in many essential oils but are mostly obtained from turpentine, obtained by the dry distillation of wood or other dry botanical material). The α-pinene obtained in North American oils is largely dextrorotary, whereas the European oils are levorotary. The majority of β-pinene, irrespective of its origin, is levorotary. As examples, α-pinene and β-pinene are found in cedar wood oil, orange oil, mandarin peel oil and in many fragrances.

Limonene ($C_{10}H_{16}$; mol mass 136.24) is a cyclic monoterpene characterized by its strong citrus smell. It is a chiral molecule, and biological sources (mainly citrus fruit) produce D-limonene ((+)-limonene), which is the (R)-enantiomer (1-methyl-4-(1-methylethenyl)-cyclohexene).

Limonene racemizes to dipentene at 300 °C. When warmed with mineral acid, limonene isomerizes to the conjugated diene α-terpinene.

Biosynthetically, limonene is formed from geranyl pyrophosphate, via cyclization of a neryl carbocation or its equivalent. The final step involves loss of a proton from the cation to form the alkene.

Alloocimene ($C_{10}H_{16}$; mol mass 136.24; also referred to as allocymene) is the acyclic monoterpene 2,6-dimethyl-2,4,6-octatriene, as described for example in Milks et al., J. Org. Chem. 1965, 30(3) 888-891. The compound includes the stereoisomeric forms. 2,4,6-octatriene, 2,6-dimethyl-,(E,E)-; (4Z,6z)-2,6-dimethyl-2,4,6-octatriene; (4Z,6e)-2,6-dimethyl-2,4,6-octatriene; 2,4,6-octatriene, 2,6-dimethyl-,(e,z)- and 2,6-dimethyl-octa-2,4,6-triene, cis.

Epoxidized poly(alloocimene) and use thereof as a cross-linker (curing agent) for polyester, polyether and polyurethane coating compositions is described in U.S. Patent No. 4,690,982. Halogenated poly(alloocimene) and use thereof in coatings as a barrier resin is described in U.S. Patent No. 4,694,047.

Geranyl acetate ($C_{12}H_{20}O_2$; mol mass 196.29; also referred to as geranyl ethanoate) is the acyclic monoterpene. 3,7-dimethyl-2,6-octadiene acetate.

Geranyl acetate may be isolated from various essential oils, including ceylon citronella, palmarosa, lemon grass, petit grain, neroli, geranium, coriander, carrot and sassafras. It can be obtained by fractional distillation of essential oils, or may be prepared semi-synthetically by the simple condensation of the more common natural terpene geraniol with acetic acid.

Cyclic monoterpenes include the isomers α-phellandrene (2-methyl-5-(1-methylethyl)-1,3-cyclohexadiene) and β-phellandrene (3-methylene-6-(1-methylethyl)cyclohexene) (each $C_{10}H_{16}$ and 136.24), which may be isolated from eucalyptus oils and balsam oils respectively.

Additional isomeric cyclic monoterpenes are α-terpinene (4-methyl-1-(1-methylethyl)-1,3-cyclohexadiene); β-terpinene (4-methylene-1-(1-methylethyl)cyclohexene) and γ-terpinene (4-methyl-1-(1-methylethyl)-1,4-cyclohexadiene), each C10H16 and mol mass 136.24. α-terpinene may be isolated from various plant sources including cardamom and marjoram oils. β-terpinene has no known natural source, but may be prepared synthetically from sabinene. γ-terpinene may be isolated from various plant sources.

Polymeric and oligomeric monoterpenes

Oligomeric monoterpenes described herein are not part of the scope of the invention.

Polymeric monoterpenes refer to a polymer compound, or a mixture of polymers of different molecular weights, which are formed from at least 6 monomeric monoterpene subunits. Oligomeric monoterpenes refer to oligomeric compounds, which are formed from 2 to 5 monomeric monoterpene subunits.

The oligomeric or polymeric monoterpene may be a synthetic product, produced by a chemical process using as a substrate a monomeric form of a specific monoterpene, for example alloocimene, limonene, polymeric α-pinene, β-pinene, geranyl acetate, α-phellandrene, γ-terpinene, 3-carene or 2-carene, as described herein. The monomeric substrate material may be isolated from a plant or plant product such as an oil, or may be chemically or enzymatically converted from a precursor terpene, as is known in the art.

An isolated fraction of oligomeric or polymeric monoterpene material may be obtained as the purified product of a chemical synthesis reaction, as exemplified in Examples 1-10.

Suitable chemical synthesis reactions include for example, an anionic polymerization reaction, a cationic polymerization reaction, a radical polymerization, a metal-catalyzed polymerization, a transition metal catalyzed polymerization and a photopolymerization reaction.

In a particular embodiment, the chemical synthesis is an anionic polymerization reaction. The anionic polymerization reaction may comprise use of butyl lithium as the catalyst.

In a particular embodiment, the chemical synthesis is a cationic polymerization reaction. The cationic polymerization reaction may comprise use of a Lewis acid as the catalyst. Suitable Lewis acids include aluminium chloride ($AlCl_3$), bismuth chloride ($SbCl_3$), tin (IV) chloride ($SnCl_4$), boron trifluoride etherate ($BF.Et_2O$) titanium (IV) chloride ($TiCl_4$), or any combination thereof.

In a particular embodiment, the chemical synthesis is a radical polymerization. The radical polymerization may comprise use of any of light, heat and a radical initiator as an initiator. In a particular embodiment, the radical polymerization comprises an initiation step wherein light is used as the initiator. In a particular embodiment, heat is used as the initiator in the initiation step. In a particular embodiment, a radical initiator is used as the initiator in the initiation step. In a particular embodiment, both light and a radical initiator are used as initiators in the initiation step. In a particular embodiment, both heat and a radical initiator are used as initiators in the initiation step. In a particular embodiment, all of light, heat and a radical initiator are used as initiators in the initiation step.

The radical initiator may be an organic or inorganic compound, for example benzoyl peroxide, 2,2'-azobisisobutyronitril (AIBN), hydrogen peroxide or potassium peroxysulfate. In a particular embodiment, the radical initiator is benzoyl peroxide. In a particular embodiment, the radical initiator is benzoyl peroxide combined with heating. In a particular embodiment, the radical initiator is benzoyl peroxide combined with heating and light. In a particular embodiment, the radical initiator is benzoyl peroxide combined with light.

In a particular embodiment, the chemical synthesis is a metal-catalyzed polymerization and/or oligomerization reaction. The metal-catalyzed polymerization and/or oligomerization may comprise use of a transition metal catalyst. It may further comprise the use of hydrogen peroxide as the initiator.

In some preferred embodiments, the chemical synthesis is a photopolymerization reaction comprising use of an energy source for providing the initiation step. Suitable energy sources include sunlight, a UV lamp, a visible light lamp. In another embodiment, a source of gamma radiation is used to initiate oligomerization and/or polymerization. In another embodiment, a combination of light and gamma radiation is used as an initiator.

Chemically synthesized oligomeric or polymeric monoterpenes, for example polymeric pinene or polymeric alloocimene, may be isolated from unreacted substrate and other reagents, analyzed and further fractionated according to molecular weight using analytical and separation methods as are known in the art. Such methods include those which separate molecules on the basis of size, charge or hydrophobicity, including for example, size exclusion chromatography (SEC), high pressure liquid chromatography (HPLC), gas liquid chromatography (GLC) and combinations thereof. Analytical methods for determining the precise chemical structure of the obtained polymer include nuclear magnetic resonance (for example $^1$NMR and $^{13}$NMR) and gas chromatography-mass spectrometry (GCMS). The same methods and approaches may be used for purifying and characterizing polymeric monoterpenes isolated from plants.

In a preferred embodiment, a fraction of polymeric alloocimene which is a product of a chemical synthesis is substantially devoid of monomers. Such a fraction may be used directly, or further purified, characterized and/or fractionated using means known in the art. Monomeric forms of terpenes and other volatile molecules can be removed by evaporation.

Non-polar solvents suitable for use in separation, purification and analysis include for example dichloromethane, hexane, tetrahydrofuran, and combinations thereof. For preparation of a composition for therapeutic use, suitable non-polar hydrophobic solvents include pharmaceutically acceptable oils as described herein.

Oligomeric and polymeric forms of the monoterpenes may be obtained using polar solvent extraction, such as with ethanol and methanol. The monomeric monoterpenes will dissolve in the polar solvent, leaving the oligomeric and polymeric forms in isolated form.

In one currently preferred embodiment, the degree of polymerization of the polymeric monoterpene is at least about 6, for example in the range from about 6 to about 200. In a particular embodiment, the degree of polymerization is at least about 25. In a particular embodiment, the polymeric material has a degree of polymerization in the range from about 6 to about 50. Suitable exemplary ranges include about 30 to about 100, or about 50 to about 150. In a particular

embodiment, the polymeric monoterpene has a number average molecular weight of at least about 1000. In a particular embodiment, the polymeric monoterpene has a number average molecular weight of up to about 25,000.

The number average molecular weight of the polymeric monoterpene is preferably at least about 3000, and even more preferably, the number average molecular weight is at least about 5000. In a particular embodiment, the polymeric material has a number average molecular weight in the range from at least about 1000 to about 25,000. In particular embodiments, the number average molecular weight is in a range selected from the group consisting of: at least about 1000 to about 5000; at least about 1000 to about 15,000; about 5000 to about 15,000; about 5000 to about 20,000; about 15,000 to about 25,000; and combinations thereof. In a particular embodiment, the polymeric monoterpene has a molecular distribution of less than 5.

The molecular weight of the polymeric monoterpene may be expressed in a number of ways, for example, weight average molecular weight or number average molecular weight, as is known in the art. Molecular weight may be determined by any of a number of means, such as light scattering, small angle neutron scattering, X-ray scattering, sedimentation velocity, viscometry (Mark-Houwink equation) and gel permeation chromatography.

The polymeric monoterpene, for example limonene, may exist as different geometric isomers, resulting from the arrangement of substituents around the carbon-carbon double bond. Such isomers are designated as the cis- or trans- configuration (also referred to respectively as the Z or E configuration), wherein cis- (or Z) represents substituents on the same side of the carbon-carbon double bond, and trans- (or E) represents substituents on opposite sides of the carbon-carbon double bond. The various geometric isomers and mixtures thereof are included within the scope of the invention. In a particular embodiment, the composition comprises a plurality of geometric isomers.

The polymeric monoterpene product may contain one or more asymmetric carbon atoms and may therefore exhibit optical isomerism and/or diastereoisomerism. All stereoisomers and diastereoisomers are included within the scope of the invention, either as a single isomer or as a mixture of sterochemical isomeric forms. The various stereoisomers and diastereoisomers may be separated using conventional techniques, for example chromatography or fractional crystallisation. Alternatively desired optical isomers may be made by reaction of the appropriate optically active starting materials under conditions which will not cause racemisation or epimerisation, or by derivatisation, for example with a homochiral acid followed by separation of the diastereomeric derivatives by conventional means. In a particular embodiment, the composition comprises a plurality of stereoisomers and/or diastereoisomers.

In particular embodiments, the polymeric monoterpene has a linear conformation, a branched conformation or a cyclic conformation.

Futhermore, herein described is at least least one oligomeric form of the subject monoterpenes. Oligomeric forms inclue dimers, trimers, tetramers and pentamers. The compositions may further include a combination of such oligomers, either from the same monoterpene or a mixture of oligomers formed from different monoterpenes.

The oligomeric form may be a combination of dimers and trimers.

Oligomeric monoterpenes include oligomeric alloocimene, oligomeric limonene, oligomeric $\alpha$-pinene, oligomeric $\beta$-pinene, oligomeric geranyl acetate, oligomeric $\alpha$-phellandrene, oligomeric $\gamma$-terpinene, oligomeric 3-carene, oligomeric 2-carene.

The oligomeric monoterpene may be selected from ologomeric alloocimene and oligomeric limonene.

Pharmaceutical compositions and modes of administration

The composition for use in the invention comprises a therapeutically effective amount of polymeric monoterpene, and a pharmaceutically acceptable carrier. The carrier is preferably lipophilic.

A suitable carrier comprises an oil, such as for example a mineral oil, a vegetable oil or combinations thereof.

The term "mineral oil" refers to a clear colorless nearly odorless and tasteless liquid obtained from the distillation of petroleum. It may also be referred to as white oil, white mineral oil, liquid petrolatum, liquid paraffin or white paraffin oil. In accordance with a particular embodiment of the invention, the mineral oil is light mineral oil, a commercially available product which may be obtained either as a NF (National Formulary) grade product or as a USP (US Pharmacopoeia) grade product. For use in the invention, the mineral oil is preferably free of aromatics and unsaturated compounds.

Suitable vegetable oils include, but are not limited to almond oil, canola oil, coconut oil, corn oil, cottonseed oil, grape seed oil, olive oil peanut oil, saffron oil, sesame oil, soybean oil, and combinations thereof. In a particular embodiment, the mineral oil is light mineral oil.

The pharmaceutically acceptable carrier may alternately or in addition comprise a suitable oil replacement. Oil replacements include alkanes having at least 10 carbon (e.g., isohexadecane), benzoate esters, aliphatic esters, noncomodogenic esters, volatile silicone compounds (e.g., cyclomethicone), and volatile silicone substitutes. Examples of benzoate esters include $C_{12}C_{15}$ alkyl benzoate, isostearyl benzoate, 2-ethyl hexyl benzoate, dipropylene glycol benzoate, octyldodecyl benzoate, stearyl benzoate, and behenyl benzoate. Examples of aliphatic esters include $C_{12}C_{15}$ alkyl octonoate and dioctyl maleate. Examples of noncomodogenic esters include isononyl isononanoate, isodecyl isononanoate, diisostearyl dimer dilinoleate, arachidyl propionate, and isotridecyl isononanoate. Examples of volatile silicone

substitutes include isohexyl decanoate, octyl isononanoate, isononyl octanoate, and diethylene glycol dioctanoate.

Cyclomethicone is an evaporative silicone which may be included in the carrier to assist in making the composition amenable to ejection from a spray dispenser. Furthermore, due to its evaporative property, cyclomethicone may assist in retaining and fixing the formulation on the surface to which it is sprayed e.g. a wound site.

The pharmaceutical composition may be formulated for administration in any of a number of forms such as for example, a capsule (including a softgel capsule), a tablet, a gel, a suppository, a suspension, a spray, a film, or an ointment. The formulations may further be in the form of one or more of a solution, a liposome, an emulsion, a microemulsion, a cement, or a powder. Preferably the oligomers and polymers are formulated by a process which protects against and/or minimizes any of oxidation, reduction or precipitation.

The pharmaceutical compositions of the invention may be administered by any means that achieve their intended purpose. For example, administration may be by topical, intramuscular, intravenous, intraperitoneal, subcutaneous, intradermal, ectodermal, mesodermal, entodermal, vaginal, rectal, intrauterine, intraurethral, intracardial, intracranial, intranasal, intrapulmonary, intrathecal, intraocular, intrarenal, intrahepatic, intratendon and auricular.

The administering may in addition comprise a technique or means such as electroporation, or sonication in order to assist in their delivery, for example transdermally. Oral administration may encompass use of a liposome protected formulation as described above. Other techniques which may be employed include for example, radio frequency or pressurized spray application.

The dosage administered will be dependent upon the age, health, and weight of the subject, the use of concurrent treatment, if any, frequency of treatment, and the nature of the effect desired. The amount of the polymeric monoterpene of the present invention in any unit dosage form comprises a therapeutically effective amount which may vary depending on the recipient subject, route and frequency of administration.

In general, the amount of polymeric monoterpene present in the pharmaceutical composition may conveniently be in the range from about 0.001% to about 12% on a weight per weight basis, based on the total weight of the composition. For topical use, the percentage of polymeric monoterpene in the composition may be in the range from about 0.05% to about 10%. For administration by injection, the percentage of polymeric monoterpene in the composition may be conveniently in the range from about 0.1% to about 7%. For oral administration, the percentage of polymeric monoterpene in the composition may be in the range from about 0.005% to about 10%.

The pharmaceutical compositions of the invention may be manufactured in a manner which is itself known to one skilled in the art, for example, by means of conventional mixing, granulating, dragee-making, softgel encapsulation, dissolving, extracting, or lyophilizing processes. Thus, pharmaceutical compositions for oral use may be obtained by combining the active compounds with solid and semi-solid excipients and suitable preservatives, and/or antioxidants protected from reactive gases.

In soft capsules, the active compounds are preferably dissolved or suspended in suitable lipids, such as fatty oils, or liquid paraffin or semisolid paraffins, waxes and a combination thereof. In addition, stabilizers and antioxidants may be added.

The carrier preferably comprises a lipid-based carrier. For example, the composition may be in the form of an emulsion or a microemulsion, based on polar lipids and surfactants. Absorption enhancers may further be included.

Oil-in-water (o/w) emulsions are commonly formed from oil(s), surfactant(s), and an aqueous phase. Oils suitable for use in typical emulsions include mineral, vegetable, animal, essential and synthetic oils, or mixtures thereof. In many cases oils rich in triglycerides, such as safflower oil, cottonseed oil, olive-oil or soybean oil are used. In its simplest form, a triglyceride-containing formulation suitable for delivering a therapeutic agent is an oil-in-water emulsion containing the therapeutic agent. Such emulsions contain the therapeutic agent solubilized in an oil phase that is dispersed in an aqueous environment with the aid of a surfactant or a combination of surfactants. Therefore, one approach is to solubilize a therapeutic agent in an oil and to disperse this oil phase in an aqueous solution. Depending on whether an oil is a solid or liquid at the ambient temperature, the oil-in-water emulsion can be characterized as a solid lipid particulate. Surfactants are also required to form solid emulsions. In order to avoid the precipitation of a drug at the lipid/water interface, the dispersion may be stabilized by emulsifying agents and provided in emulsion form. Drugs dissolved in the oil phase or the solid lipid core phase may be dispersed by mechanical force to create droplets or spheres suspended in the aqueous phase that are stable in storage as a pharmaceutical preparation.

The formation of a stable oil-in-water emulsion may be enhanced by the use of surfactants that form the interface between the strictly hydrophobic oil and water. Depending on the nature of the oil and one or more surfactants, either large droplets characteristic of oil-in-water emulsions or much smaller structures characteristic of microemulsions or micellar structures are formed. Further control over size of droplets or particles can be obtained by high pressure homogenization or similar shear forces. Lipid particles are typically formed at higher ambient temperatures to melt the hydrophobic components.

Microemulsion systems are ternary or quaternary systems typically formed from an oil phase, a surfactant, and water. For example, U.S. Pat. No. 5,707,648 describes microemulsions that contain an oil phase, an aqueous phase, and a mixture of surfactants. Microemulsions are thermodynamically stable, such that the droplets will not coalesce and

precipitate over time. The diameter of microemulsion droplets is in the range of 10 to 200 nanometers, while emulsion droplets are generally greater than a micron.

Carriers suitable for formulating compositions comprising monoterprenes have been described, for example in US Patent Application Publication No. 2006/0104997.

Other pharmaceutical compositions for oral use include a film designed to adhere to the oral mucosa, as disclosed for example in U.S. Patent Nos. 4,713,243; 5,948,430; 6,177,096; 6,284,264; 6,592,887, and 6,709,671.

Pharmaceutical compositions in the form of suppositories consist of a combination of the active compound(s) with a suppository base. Suitable suppository bases include for example, natural or synthetic triglycerides, polyethylene glycols, or paraffin hydrocarbons.

Formulations for parenteral administration include suspensions and microparticle dispersions of the active compounds as appropriate. In a particular embodiment, oily injection suspensions may be administered. Suitable lipophilic solvents or vehicles include fatty oils, e.g., sesame oil, or synthetic fatty acid esters, e.g., ethyl oleate, triglycerides, polyethylene glycol-400, cremophor, or cyclodextrins. Injection suspensions may contain substances which increase the viscosity of the suspension include, e.g., sodium carboxymethyl cellulose, sorbitol, and/or dextran. Optionally, the suspension may also contain stabilizers.

Pharmaceutical compositions can also be prepared using liposomes comprising the active ingredient. As is known in the art, liposomes are generally derived from phospholipids or other lipid substances. Liposomes are formed by mono- or multi-lamellar hydrated liquid crystals which are dispersed in an aqueous medium. Any non-toxic, physiologically acceptable and metabolisable lipid capable of forming liposomes can be used. In general, the preferred lipids are phospholipids and the phosphatidyl cholines (lecithins), both natural and synthetic. Methods to form liposomes are known in the art, as disclosed for example, in Prescott, Ed., Methods in Cell Biology, Volume Antioxidants may also be included, as well as agents imparting color or fragrance, if desired.. Ointments may be formulated for example, by mixing a solution of the active ingredient in a vegetable oil such as almond oil with warm soft paraffin, and allowing the mixture to cool.

The pharmaceutical composition may be formulated in the form of a glue, such as those comprising octocyanoacrylate used for wound closure applications. These steps are taken after the polymer monoterpene material has been already protected by a hydrophobic lipophilic surrounding which forms a barrier between the active polymer and the additional excipients desired in the formula.

<u>Therapeutic uses</u>

The present invention provides compositions for use in treating impaired neurological function in a subject in need thereof. The uses comprise administering to the subject a therapeutically effective amount of a composition comprising polymeric monoterpenes, as described herein.

Further described is a method of treating of treating a skin or scalp disorder, comprising topically administering to the subject a therapeutically effective amount of a composition comprising oligomeric or polymeric monoterpenes, as described herein.

Further described is a composition for use in inducing a regenerative process in an animal, comprising administering to the animal a therapeutically effective amount of a composition comprising polymeric monoterpenes, as described herein.

The step of administering the composition may comprise any acceptable route including oral, topical, intramuscular, intravenous, intraperitoneal, subcutaneous, intradermal, ectodermal, mesodermal, entodermal, vaginal, rectal, intrauterine, intraurethral, intracardial, intracranial, intramyocardial, intranasal, intrapulmonary, intrathecal, intraocular, intrarenal, intrahepatic, intratendon and auricular.

In particular embodiments, the step of administering comprises contacting cells of a particular type, of a particular lineage or at a particular stage of differentiation, with the composition. The cells may be any of a wide variety of cell types, including in particular, neural cells, neuronal cells, endothelial cells and epithelial cells. Further, the cells may be of any lineage for example, ectodermal, mesodermal and entodermal lineages. In various embodiments, the step of contacting cells is carried out *in vivo, ex vivo* or *in vitro.*

The method disclosed herein for treating impaired neurological function is particularly advantageous for subjects afflicted with neurodegenerative conditions and diseases, including in particular, vascular dementia, senile dementia, Alzheimer's disease, schizophrenia, amyotrophic laterial sclerosis (ALS), multiple sclerosis and Parkinson's disease. In other cases, the method may be advantageously applied in subjects suffering from impaired neurological function due to an infection (e.g. viral, bacterial, fungal, parasitic) or an immunological disorder. In a particular embodiment, the impaired neurological function is due to exposure to a drug, such as an anesthetic.

Skin and scalp disorders include all disorders of skin, scalp and hair appendages, including for example, nails and hair follicles. Particular conditions which may benefit from the invention include allopecia,( eczema, psoriasis, acne, vitiligo) seborrheic keratosis, seborrhea and skin wounds. Skin wounds include venous leg ulcers, pressure ulcers,

diabetic foot ulcers, burns, amputation wounds, decubitus ulcers (bed sore), split-skin donor grafts, skin graft donor sites, medical device implantation sites, bite wounds, frostbite wounds, puncture wounds, shrapnel wounds, dermabrasions, an infection wounds and surgical wounds. Wounds may be the result of infection; exposure to ionizing radiation; exposure to laser, or exposure to a chemical agent.

The described compositions may be particularly effective and economical for treatment of chronic non-healing wounds. As is known to one of ordinary skill in the art, non-healing wounds are distinguished by various criteria, including the rate of closure measured by length, width and depth of the wound over time.

The step of contacting cells may be carried out *in vitro* or *ex vivo.* In particular, cells, or an organ or tissue derived there from which is intended for implantation or transplantation into the subject may be treated according to the invention. For example, cell explants or cells or tissues grown and maintained in culture may be contacted with the composition. The cells may originate for example, from stem cells of an autologous or homologous donor, and be intended for organ regeneration and/or implantation into a recipient. In other cases, the cells are from a heterologous donor and are intended for implantation or transplantation into a recipient. In a particular embodiment, the cells are those of an organ or tissue from a heterologous donor intended for implantation or transplantation into a recipient. In a particular embodiment, the cells are those which secrete soluble factors.

The medical use may be carried out prior to or following implantation of a medical device into the subject. Medical devices include, but are not limited to a prosthetic, an artificial organ or component thereof, a valve, a catheter, a tube, a stent, an artificial membrane, a pacemaker, a sensor, an endoscope, an imaging device, a pump, a wire and an implant. Implants include, but are not limited to a cardiac implant, a cochlear implant, a corneal implant, a cranial implant, a dental implant, a maxillofacial implant, an organ implant, an orthopedic implant, a vascular implant, an intraarticular implant and a breast implant.

In a particular embodiment, the medical device is an organ implant, which may in certain cases comprise autologous cells of the subject.

In a particular embodiment, the step of contacting comprises a means selected from the group consisting of electroporation, sonication, radio frequency, pressurized spray and combinations thereof.

In a particular embodiment, the step of contacting comprises establishing contact between interstitial fluid or a cell surface and the composition. This may be particularly advantageous for wounds which are surrounded by interstitial fluid. Contact between interstitial fluid and the composition may be accomplished by piercing and/or teasing the dermis with a needle, a microneedle, or an apparatus comprising a plurality of needles or microneedles. Such needles or microneedles are preferably non-hollow and may be fashioned in a plurality for example, on a comb or brush-like apparatus.

The uses of the invention are suitable for application in humans, non-human mammals, and non-mammalian subjects such as fish and birds.

Articles of manufacture

The invention may encompass use of an article of manufacture which incorporates the composition comprising polymeric monoterpenes described herein.

The pharmaceutical composition may be in the form of a coating on the article of manufacture, or may be contained within a vessel which is integral to the article of manufacture. The pharmaceutical composition is advantageously present as a coating on devices which are inserted to the body and are intended for integration therein, for example an implant. The pharmaceutical composition can thus promote tissue closure over the implant due to the activity of polymeric monoterpenes in inducing cell differentiation or regeneration leading to repair of the atrophic tissue.

The pharmaceutical composition may be advantageously incorporated onto or into articles used in wound healing, for example, a dressing or bandage. The pharmaceutical composition can thus promote wound healing due to the activity of polymeric monoterpene in inducing cell differentiation.

In other cases, the pharmaceutical composition may be incorporated to a delivery device such as a needle, an injection device or a spray dispenser from which the composition is delivered to a body site requiring therapy, for example a wound site.

Articles of manufacture include, but are not limited to a fabric article, a diaper, a wound dressing, a medical device, a needle, a microneedle, an injection device and a spray dispenser. In a particular embodiment, the article of manufacture comprises a plurality of microneedles. Medical devices and implants are as hereinbefore described.

The following examples are presented in order to more fully illustrate certain embodiments of the invention. They should in no way, however, be construed as limiting the broad scope of the invention. One skilled in the art can readily devise many variations and modifications of the principles disclosed herein.

EXAMPLES

Example 1. Base initiated polymerization of acyclic monoterpenes.

The synthetic reaction used for polymeric acyclic monoterpenes, such as alloocimene involves a mechanism of anionic polymerization (known as the "Michael reaction"), represented by the following scheme:

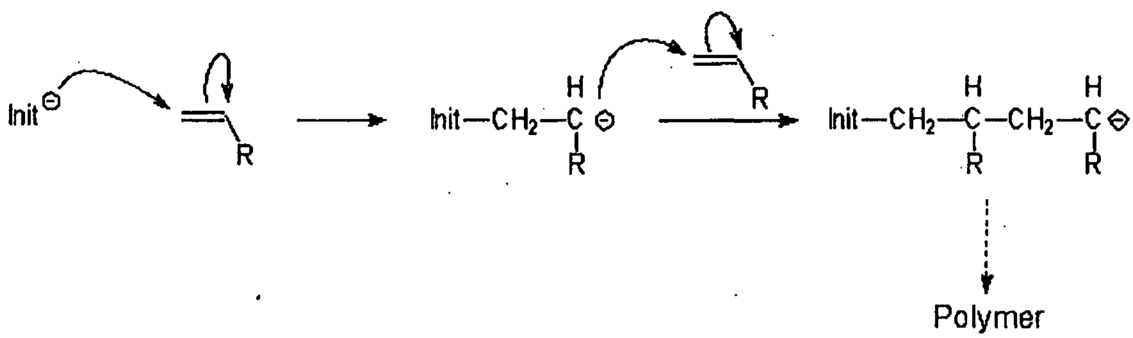

For initiation to be successful, the free energy of the initiation step must be favorable. Therefore, it is necessary to match the monomer with the appropriate strength of initiator so that the first addition is "downhill". A typical anionic reaction is the polymerization of styrene using butyllithium, $C_4H_9Li$, in an inert solvent such as *n*-hexane.

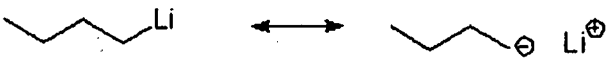

Strong, initiates styrene, isoprene.

When carried out under the appropriate conditions, termination reactions do not occur in anionic polymerization. One typically adds a compound such as water or alcohol to terminate the process. The new anionic species is too weak to reinitiate, as shown in the following scheme.

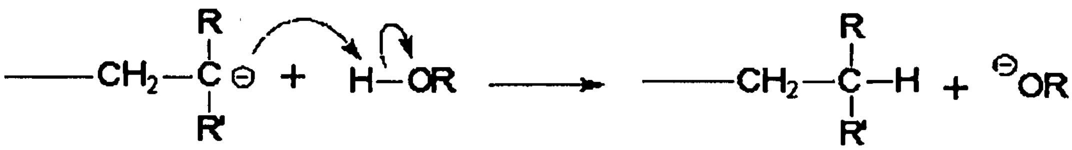

Compounds such as water, alcohols, molecular oxygen, carbon dioxide, etc react very quickly with the carbanions at the chain ends, terminating the propagation. Since the chain ends are relatively few in number only a very small amount of water need be present to terminate the polymerization reaction. Termination does not occur by polymer-polymer interaction.

Anionic polymerization gives rise to very sharp molecular mass distributions because transfer processes are absent. If the solvent is extremely pure, the polymer chains will still be active after all the monomer has been consumed.

The degree of polymerization is simply:

$$n = \frac{[M]}{[I]}$$

As indicated above, butyl lithium is an appropriate initiator for anionic polymerization for isoprene-containing molecules such as terpenes. Therefore, it has been used in the synthesis of the polymers described herein. Advantageously, the above described procedure does not require any kind of work up aside from evaporation and solvent replacement.

While the above described procedure is generally disclosed in the prior art (see for example Newmark et al (1988) J. Polym Sci.26:71-77), important modifications disclosed herein are the work up in a high dilution of hexane and the final step of changing the solvent to oil, in order to obtain neat polymer which retains its biological activity with high potency.

Example 2. Acid-catalyzed polymerization of α-pinene.

Toluene (20ml), $AlCl_3$ (150mg, 1.2 mMol) and $SbCl_3$ (121mg, 0.6mMol) were added to a 3-necked flask equipped with condenser and under a $N_2$ atmosphere,. The reaction mixture was cooled to -15 °C. Then 5g (0.36mol) of α-pinene was added. The reaction was stirred for 4h at -30 °C. The catalyst was filtered off and the solvent and other volatiles were evaporated by applying vacuum. SEC analysis indicated that the obtained polymer product had a molecular weight in the range of 10-0.5 kDa.

Example 3. Acid-catalyzed polymerization of alloocimene.

Toluene (20ml) and boron trifluoride etherate ($BF_3{\cdot}O(Et)_2$; 0.1ml 0.8 mMol) was added to a 3-necked flask equipped with condenser and under a $N_2$ atmosphere. The reaction mixture was cooled to -30 °C. Then 5g (36mmol) of alloocimene was added. The reaction was stirred for 4h at -30 °C. Insoluble material was filtered off and the solvent and other volatiles were evaporated by applying vacuum. SEC analysis (Figure 5) indicated that the obtained polymer product had a molecular weight in the range of 2-10 kDa.

Example 4. Photopolymerization of geranyl acetate using benzoyl peroxide.

Heptane (20ml) and benzoyl peroxide (1 mMol) were added to a 3-necked flask equipped with condenser and under a $N_2$ atmosphere. Then 5g (25mmol) of geranyl acetate was added. The reaction was heated to reflux for 4h. Insoluble material was filtered off and the solvent and other volatiles were evaporated by applying vacuum. SEC analysis indicated that the obtained oligomeric product had a molecular weight in the range of 0.3-0.5 kDa.

Example 5. Acid catalyzed polymerization of limonene.

Toluene (20ml) and $BF_3{\cdot}O(Et)_2$ (0.1ml 0.8 mMol) was added to a 3-necked flask equipped with condenser and under a $N_2$ atmosphere. The reaction mixture was cooled to-70 °C. Then 5g (36mmol) of limonene was added. The reaction was stirred for 4h at -70 °C. Insoluble material was filtered off and the solvent and other volatiles were evaporated by applying vacuum. SEC analysis (Figure 6) indicated that the obtained polymer product had a molecular weight in the range of 2-5 kDa.

Example 6. Acid catalyzed polymerization of β-pinene.

Toluene (20ml), $AlCl_3$ (0.8 mMol) and $SbCl_3$ (0.4 mMol) were added to a 3-necked flask equipped with condenser and under a $N_2$ atmosphere. The reaction mixture was cooled to -20 °C. Then 5g (36mmol) of α-pinene was added. The reaction was stirred for 4h at -20 °C. Insoluble material was filtered off and the solvent and other volatiles were evaporated by applying vacuum. SEC analysis indicated that the obtained product was a mixture of oligomers and polymer of molecular weight in the range of 0.5-4.5 kDa.

Example 7. Acid catalyzed polymerization of γ-terpinene.

Toluene (20ml) and $SnCl_4$ (1 mMol) were added to a 3-necked flask equipped with condenser and under a $N_2$ atmosphere. The reaction mixture was cooled to -20 °C. Then 5g (36mmol) of γ-terpinene was added. The reaction was stirred for 4h at -20 °C. Insoluble material was filtered off and the solvent and other volatiles were evaporated by applying vacuum. SEC analysis indicated that the obtained mixture of oligomers had a molecular weight of about 0.5 kDa.

Reference Example 8. Acid catalyzed oligomerization of 3-carene.

Toluene (20ml) and $AlCl_3$ (1 mMol) were added to a 3-necked flask equipped with condenser and under a $N_2$ atmosphere. The reaction mixture was cooled to -30 °C. Then 5g (36mmol) of 3-carene was added. The reaction was stirred for 4h at -30 °C. Insoluble material was filtered off and the solvent and other volatiles were evaporated by applying vacuum. SEC analysis indicated that the obtained product was aa mixture of dimers and trimers.

Example 9. Acid catalyzed polymerization of α-phellandrene.

Toluene (20ml) and $AlCl_3$ (1.2 mMol) were added to a 3-necked flask equipped with condenser and under a $N_2$ atmosphere. The reaction mixture was cooled to -70 °C. Then 5g (36mmol) of α-phellandrene was added. The reaction was stirred for 4h at -70 °C. Insoluble material was filtered off and the solvent and other volatiles were evaporated

by applying vacuum. SEC analysis indicated that the obtained polymer product had a molecular weight of about 1 kDa.

Example 10. Polymerization of γ-terpinene using benzoyl peroxide as the radical initiator.

Heptane (20ml) and benzoyl peroxide (1 mMol) were added to a 3-necked flask equipped with condenser and under a $N_2$ atmosphere. Then 5g (36mmol) of γ-terpinene was added. The reaction was heated to reflux for 4h. Insoluble material was filtered off and the solvent and other volatiles were evaporated by applying vacuum. SEC analysis indicated that the obtained oligomeric procduct had a molecular weight in the range of 0.4-0.5 kDa.

Example 11. Polymeric forms of limonene and of alloocimene induce neuronal-like differentiation in retinal pigment epithelial cell cultures.

Overview

The present invention is directed to induction of differentiation and cell maturation, and has direct application to regeneration of functional tissue, in particular neuronal tissue. Our experimental findings show that the polymeric terpenes tested, including limonene, alloocimene, pinene and geranyl, induce differentiation of retinal pigment epithelial cells, an epithelial tissue of neuronal origin, to morphological neuronal cells producing axons, dendrites and junctions between cells known as synapses. The morphological differentiation in treated cells is accompanied by de novo expression of the neuron-specific differentiation antigen β3 tubulin. The induction of neuronal cell differentiation strongly suggests that the polymers affect neuronal stem cell differentiation into functional neurons.

Current dogma on the pathology of dementia and Alzheimer's disease holds that the deficiency involves the failure of neurons to form functional synaptic junctions (Kimura R, Ohno M. Impairments in remote memory stabilization precede hippocampal synaptic and cognitive failures in 5XFAD Alzheimer mouse model. Neurobiol Dis. 2008 Nov 5).

Accordingly, the experiments described herein support use of polymeric terpenes, as a therapeutic modality to elicit neuro-regeneration in neurodegenerative diseases such as dementia and Alzheimer's disease.

Retinal pigment epithelium (RPE) cells

Studies aimed at evaluating effects polymeric monoterpens on various cell lines of human origin led to use of ARPE-19 cells, a non-malignant human retinal pigment epithelial cell line.

The retinal pigment epithelium (RPE) is a single layer of hexagonal pigmented epithelial cells of neuronal origin, which forms the outermost cell layer of the eye retina and is attached to the underlying choroid. RPE functions include support, nourishment and protection of the underlying photoreceptors of the neuro-retina.

RPE cells are involved in the phagocytosis of the outer segment of photoreceptor cells, in the vitamin A cycle where they isomerize all-trans retinol to 11-is retinal and in supplying the photoreceptors with D-glucose, amino acids and ascorbic acid.

Although *in vivo* the RPE is pigmented, ARPE-19 cells do not form melanin and are not pigmented. In culture the cells grow as spindle shaped and as polygonal cells.

Methods

ARPE-19 cells (obtained from the American Type Culture Collection, ATCC) were plated in flat bottom 96 well tissue culture microplates (Costar) at a concentration of 2 - $5 \times 10^3$ cells per well (1 - $2.5 \times 10^4$ cells/mL) in a growth medium consisting of DMEM:Ham F-12, 1:1, supplemented with 10% Fetal Bovine Serum, 200 mM glutamine, 100 units/mL penicillin and 100 μg/mL streptomycin. The cells were allowed to adhere to the plate surfaces overnight prior to treatment with polymeric monoterpene.

Each type of polymeric monoterpene was synthesized to provide a 10% solution in grape seed oil, olive oil, Mygliol 810 or Mygliol 812. The preparations were added to the cultures at volumes of 0.5 μl, 2 μl, 5 μl and 20 μl. These volumes, introduced into an overall sample medium volume of 200 μl, correspond to final alloocimene concentrations of 0.025%, 0.1%, 0.25% and 1%, respectively. The oil carrier served as a vehicle control and was applied to control cultures at the same volumes.

The cultures were incubated in a 37 °C, 5% $CO_2$ incubator for 72 hrs. The medium was then removed, the cultures washed twice with phosphate buffered saline (PBS), fixed with absolute methanol for 10 min and stained with Hemacolor® reagents (Boehringer Mannheim), which stain cells in a manner similar to Giemsa, and may beused in a quantitative cell viability assay (see Keisari, Y. A colorimetric microtiter assay for the quantitation of cytokine activity on adherent cells in tissue culture. J. Immunol. Methods 146, 155-161, 1992). The degree of differentiation was determined by optical microscope.

Treatment of ARPE-19 RPE cells with the polymeric monoterpenes was unexpectedly found to induce dramatic morphological changes that are unequivocally characteristic of neuro-differentiation. The morphological changes did not occur in control cultures treated with oil carrier alone, regardless of the oil used as the carrier for the active compound. The morphological changes were also associated with cessation in cell proliferation, further supporting the conclusion that the polymeric monoterpenes induce neuro-differentiation.

Figure 2 shows the effect of polymeric limonene on ARPE-19 RPE cells. Control oil-treated cultures displayed the typical spindle shaped and polygonal growth pattern characteristic of ARPE-19 RPE cells (Fig. 2B). After 72 hours of incubation, polymer-treated cells (0.025%; 0.25 mg/ml) displayed a larger number of thinner long protrusions reminiscent of dendrites (Fig. 2A). The thin long protrusions formed junctions with similar protrusions in adjacent cells creating a network of inter-connected cells, potentially capable of communicating information between one another. Similar networks occur normally between neurons in the central nervous system and enable transmission and processing of information.

Experiments carried out using oxidized preparations of polymeric limonene did not induce such differentiation.

Figure 3 shows the effect of polymeric alloocimene on ARPE-19 RPE cells. Fig. 3A shows that cells that were treated with polymeric alloocimene exhibited differentiation-like changes, while cells that were treated with vehicle (cottonseed oil) alone, did not exhibit such changes (Fig. 3B).

Figure 4 shows a comparison of the effects of polymeric alloocimene and monomeric alloocimene on ARPE-19 RPE cells. Fig. 4A shows that ARPE-19 RPE cells treated with polymeric alloocimene showed evidence of differentiation, whereas cells treated with monomeric alloocimene (Fig. 4B) or with cottonseed oil vehicle (Fig. 4C) showed no such effects.

<u>A scoring system for the potency of polymeric monoterpenes in inducing cell differentiation</u>

On the basis of the above results, a scoring system was developed to evaluate the potency of polymeric monoterpenes for inducing differentiation in cell culture, with cells plated $2x10^3$ per well. The grades and their respective descriptions are set out in Table 1.

Table 1.

| Effect | Grade | | | | | | | | | | | |
|---|---|---|---|---|---|---|---|---|---|---|---|---|
| Proliferation rate | High=0 Medium =1 Low=2 | 0 | 0 | 0 | 1 | 1 | 1 | 2 | 1 | 2 | 1 | 2 |
| Cells are forming elongated protrusions | No=0 protrusi ons=1 neuron like=2 | 0 | 1 | 1 | 1 | 1 | 1 | 1 | 1 | 2 | 2 | 2 |
| Neurites (neuron-like elongations)/ body ratio | ≤2=0 >2≤3=1 >3=4 | 0 | 0 | 0 | 0 | 1 | 1 | 1 | 1 | 1 | 4 | 4 |
| Percent of differentiated cells | ≤10%=0 >10%≤3 0%=1 >30%≤7 0%=2 ≥70%=3 | 0 | 0 | 0 | 0 | 0 | 1 | 1 | 2 | 2 | 2 | 3 |
| Clearly visible junctions between neurites and/or cell bodies | ≤30%,= 0 >30%<7 0%,=1 ≥70%=2 | 0 | 0 | 0 | 0 | 0 | 0 | 0 | 1 | 1 | 1 | 2 |
| Visible, clear synaptic-like boutons along the neurites and at the ends of the neurites. | <30%=0 >30%<5 0%=1 ≥70%=2 | 0 | 0 | 0 | 0 | 0 | 0 | 0 | 0 | 1 | 1 | 2 |
| **Total Differentiation Grade** | ***0*** | ***1*** | ***1*** | ***2*** | ***3*** | ***4*** | ***5*** | ***6*** | ***10*** | ***11*** | ***15*** | |
| **Differentiation Score** | ***0*** | ***1*** | ***2*** | ***3*** | ***4*** | ***5*** | | | | | | |

Reference <u>Example 12. Polymeric alloocimene induces cell differentiation followed by cell death in tumor cell lines</u>

The effects of polymeric alloocimene on two melanoma cell lines and three neuroblastoma cell lines were investigated. Human melanoma cell line 5151 and murine melanoma cell line B16F10 both proliferate in tissue culture in an undifferentiated manner and do not produce melanin. Human neuroblastoma cell lines Lan-1, Lan-5 and SY5Y

proliferate in culture as spindle shaped cells that do not exhibit differentiation morphology.

Methods

Cells were plated at $2x10^3$ cells per well in 96 well flat bottom microplates (Costar) and cultured in 200 microliters of medium DMEM (Dulbecco's medium) supplemented with 10 fetal bovine serum, 200 mM L-glutamine, 100 units/ml penicillin and 100 microgram/ml streptomycin (all reagents from Gibco-BRL). Following overnight attachment, polymeric alloocimene (from a 10% solution in grape seed oil) was added to the cell cultures to provide final concentrations of 0.025%, 0.1%, 0.25% and 0.5%, and incubation was continued for 48 and 72 hours. The grape seed oil vehicle was used as control. After 72 hours, cells were fixed with methanol and stained with Hemacolor® reagents (Boehringer Mannheim).

Results

Treatment of melanoma cells with polymeric alloocimene was found to induce formation of melanin after 24-48 hrs, as compared to the control treated cells. The polymer treatment further caused arrest of replication, as shown by the decreased cell density. By 72 hours, cell death occurred in cultures incubated with each of the four polymer concentrations tested.

Upon treatment of neuroblastoma cell lines Lan-1, Lan-5 and SY5Y with polymeric alloocimene (final concentration 0.025%), the cells began to develop dendrite-like protrusions and cell proliferation ceased. Higher concentrations caused cell death in the entire culture. Thus, the treatment with polymeric alloocimene induced morphological neuron-like differentiation features that were followed by cell death.

Conclusion

Polymeric alloocimene is effective for inducting differentiation of cell lines derived from the malignant cancers melanoma and neuroblastoma.

A block in terminal differentiation is recognized as a major avenue in the perpetuation of cell proliferation in cancer. Overcoming this block has already proven to be an effective treatment modality of several forms of cancer (e.g. retinoids in treatment of acute promyelocytic leukemia) and is now known as "targeted therapy". Targeted therapy does not kill cancerous cells but modifies their behavior, primarily by inducing differentiation. Accordingly, the aggressiveness of many cancers can be reduced.

As disclosed herein, a polymeric monoterpene was found to overcome the block in tumor cell differentiation, as indicated by formation of neuronal cell dendrites in neuroblastoma cell lines, and induction of melanin formation in melanoma cell lines. In both cases these changes were associated with cessation in cell proliferation and cell death.

Example 13. Chemically synthesized polymeric forms of alloocimene, limonene, pinene, or geranyl acetate induce cell differentiation in retinal pigment epithelial cell cultures.

Chemically synthesized forms of limonene, geranyl acetate, pinene and alloocimene were used separately. Cells were plated in flat bottom 96-well tissue culture microplates (BIOFIL) at a concentration of $5x10^3$ cells per well ($2.5x10^4$ cells/mL) in a growth medium consisting of DMEM:Ham F-12, 1:1, supplemented with 10% Fetal Bovine Serum, 200 mM glutamine, 100 units/mL penicillin and 100 $\mu$g/mL streptomycin. The cells were allowed to adhere to the plate surfaces overnight prior to treatment with the chemically synthesized polymers.

The cultures were incubated in a 37 °C, 5% $CO_2$ incubator for 72 hrs. The medium was then removed, the cultures washed twice with phosphate buffered saline (PBS), fixed with absolute methanol for 10 min and stained with Hemacolor® reagents.

Results

All the polymers above mentioned were shown to have activity in inducing neuro-differentiation in ARPE-19 cells.

Conclusion

The observed results support the conclusion that chemically synthesized polymeric monoterpenes have activity in inducing differentiation of neuronal cells.

Example 14. Comparison of polymeric allocimene to monomeric allocimene in inducing cell differentiation in retinal pigment epithelial cell cultures.

The experimental procedure was identical to that described in Example 13. Figure 4A shows that polymeric allocimene induced neuro-differentiation while monomeric allocimene (Fig. 4B) has no influence on the cells, similar to the results seen with cells that were treated with vehicle only (Fig. 4C).

The observed results support the conclusion that chemically synthesized polymeric alloocimene has activity in inducing differentiation of neuronal cells. In contrast, monomeric alloocimene does not exhibit this activity.

Reference Example 15. Wound healing in dogs.

A female dog having an open chronic wound for more than three months which resisted standard typical treatment was treated by topical treatment with synthetic polymeric alloocimene. The treatment resulted in rapid wound closure starting with rapid epithelization and formation of granulation tissue, noticible within three days. The wound was completely healed within 4 weeks. Remarkably, scar-less tissue covered the wound. Wound healing contracted inwards towards the center of the wound, suggesting the presence of fibro-myocytes (of mesodermal origin). Similar results were reported by a veterinarian who treated a non-healing wound on a horse leg. In both cases the fur around the wound started to show renewal of young fur.

In another aging male dog afflicted by allopecia, topical treatment with polymeric alloocimene resulted in re-growth of the fur to become integrated with the surrounding fur.

Reference Example 16. Treatment of wounds in fish.

Gold fish as well as koi fish (both in the carp family) are prone to integument ulcers caused by bacteria, in particular *Aeuromonas hydrophila.*

Gold fish weighing approximately 100 gram each, which had developed bacterial ulcerations, were divided into two groups in separate tanks, each group containing four fish. Each tank was filled with a volume of 100 liters of water and maintained under aeration with an air pump. The groups were randomized by weight and wound size (in the range of 0.7-1.5 cm by 0.7-1.5 cm). Each fish was injected intramuscularly through intact integument at a site approximately 5 mm from an ulcer with 10 micro liters of either grape seed oil alone (control group), or a 1% solution of each of polymeric alloocimene and limonene (as described in Examples 3 and 5) in grape seed oil (treatment group).

Fish in the test group began to improve progressively following 4 weekly cycles of treatment with the polymerized monoterpenes and were healed over a period of 4-6 weeks. All fish in this group survived through the 7 week duration of the study. These fish also exhibited alert and responsive behavior including active swimming, searching for and snatching at food provided at the water surface, and rapid, startled movement away in response to percussion on the wall of the tank.

In contrast, fish in the control group displayed no improvement in the condition of their ulcers. The fish were lethargic, exhibited sedentary behavior at the bottom of the tank, and did not respond to stimulation. All of the fish in this group died by the end of six weeks.

The differences between these two groups were highly significant in both parameters: fish survival and wound closure. In addition the treated group displayed more activity in reacting to stimulations such as alertness to food supplied and percussion on the tank wall.

## Claims

1. A pharmaceutical composition for therapeutic use in treating impaired neurological function, wherein the pharmaceutical composition comprises at least one polymeric form of a monoterpene, wherein the monoterpene is selected from the group consisting of alloocimene, limonene, α-pinene, β-pinene, geranyl acetate, 3-carene and 2-carene, and a pharmaceutically acceptable carrier; and wherein the composition is substantially devoid of the corresponding monomeric form of said monoterpene.

2. The pharmaceutical composition for use according to claim 1, wherein the impaired neurological function is associated with a condition selected from the group consisting of vascular dementia, senile dementia, Alzheimer's disease, schizophrenia, amyotrophic lateral sclerosis (ALS), Huntington's disease, multiple sclerosis and Parkinson's disease.

3. The pharmaceutical composition for use according to claim 1, wherein the composition comprises a polymeric monoterpene selected from the group consisting of polymeric alloocimene, polymeric limonene, polymeric α-pinene,

polymeric β-pinene, polymeric geranyl acetate, polymeric 3-carene, polymeric 2-carene, and combinations thereof.

4. The pharmaceutical composition for use according to claim 3, wherein the composition comprises from about 0.01 to about 12% (w/w) of said polymeric monoterpene, based on the total weight of the composition.

5. The pharmaceutical composition for use according to claim 1, wherein the composition comprises a plurality of isomers of said monoterpene, wherein the plurality of isomers is selected from geometric isomers, stereoisomers, diastereoisomers and combinations thereof.

6. The pharmaceutical composition for use according to claim 3, wherein the polymeric monoterpene has a degree of polymerization in the range of at least about 6 to about 200.

7. The pharmaceutical composition for use according to claim 6, wherein the degree of polymerization is at least about 25; or wherein the degree of polymerization is in the range of about 6 to about 50.

8. The pharmaceutical composition for use according to claim 3, wherein the polymeric monoterpene has a number average molecular weight of at least about 3000.

9. The pharmaceutical composition for use according to claim 3, wherein the polymeric monoterpene has a number average molecular weight in the range from at least about 1000 to about 25,000; preferably wherein the number average molecular weight is in a range selected from the group consisting of at least about 1000 to about 5000; at least about 1000 to about 15,000; about 5000 to about 15,000; about 5000 to about 20,000; about 15,000 to about 25,000, and combinations thereof.

10. The pharmaceutical composition for use according to claim 3, wherein the polymeric monoterpene is the product of chemical synthesis selected from the group consisting of an anionic polymerization reaction, a cationic polymerization reaction, a radical polymerization, a metal-catalyzed polymerization and a photopolymerization reaction.

11. The pharmaceutical composition for use according to claim 10, wherein the chemical synthesis comprises use of a monomeric monoterpene as a substrate, and wherein the monomeric monoterpene is selected from the group consisting of alloocimene, limonene, α-pinene, β-pinene, geranyl acetate, 3-carene and 2-carene.

12. The pharmaceutical composition for use according to claim 11, wherein the monomeric monoterpene substrate is derived from a plant species.

13. The pharmaceutical composition for use according to claim 1, wherein the composition comprises a pharmaceutically acceptable carrier comprising at least one oil or wax; preferably wherein the oil is a vegetable oil selected from the group consisting of almond oil, canola oil, coconut oil, corn oil, cottonseed oil, grape seed oil, olive oil peanut oil, saffron oil, sesame oil, soybean oil, and combinations thereof.

14. The pharmaceutical composition for use according to claim 1, wherein the pharmaceutical composition is to be administered by a route which is other than an oral or enteral route; or wherein the pharmaceutical composition is to be administered by a route selected from the group consisting of topical, intramuscular, intravenous, intraperitoneal, subcutaneous, intradermal, vaginal, rectal, intracranial, intranasal, intraocular, and auricular.

15. The pharmaceutical composition for use according to claim 1, wherein the pharmaceutical composition is to be administered by contacting cells of a subject with the pharmaceutical composition, and wherein the cells are selected from the group consisting of neural cells, neuronal cells, endothelial cells, epithelial cells, ectodermal lineage cells, mesodermal lineage cells and entodermal lineage cells; and wherein the step of contacting cells is carried out *ex vivo* or *in vitro* and said cells are thereafter transplanted or implanted into the subject.

**Patentansprüche**

1. Pharmazeutische Zusammensetzung zur therapeutischen Anwendung bei der Behandlung einer beeinträchtigten neurologischen Funktion, wobei die pharmazeutische Zusammensetzung mindestens eine polymere Form eines Monoterpens umfasst, wobei das Monoterpen ausgewählt wird aus der Gruppe bestehend aus Alloocimen, Limonen, α-Pinen, β-Pinen, Geranylacetat, 3-Caren und 2-Caren, und einem pharmazeutisch akzeptablen Träger; und wobei

die Zusammensetzung im Wesentlichen frei von der entsprechenden monomeren Form des Monoterpens ist.

2. Pharmazeutische Zusammensetzung zur Verwendung nach Anspruch 1, wobei die beeinträchtigte neurologische Funktion mit einem Zustand verbunden ist, der aus der Gruppe bestehend aus vaskulärer Demenz, seniler Demenz, Alzheimer-Krankheit, Schizophrenie, amyotrophischer Lateralsklerose (ALS), Huntington-Krankheit, multipler Sklerose und Parkinson-Krankheit ausgewählt wird.

3. Pharmazeutische Zusammensetzung zur Verwendung nach Anspruch 1, wobei die Zusammensetzung ein polymeres Monoterpen umfasst, das aus der Gruppe bestehend aus polymerem Alloocimen, polymerem Limonen, polymerem α-Pinen, polymerem β-Pinen, polymerem Geranylacetat, polymerem 3-Caren, polymerem 2-Caren und Kombinationen derselben ausgewählt wird.

4. Pharmazeutische Zusammensetzung zur Verwendung nach Anspruch 3, wobei die Zusammensetzung etwa 0,01 bis etwa 12 % (w/w) des polymeren Monoterpens umfasst, basierend auf dem Gesamtgewicht der Zusammensetzung.

5. Pharmazeutische Zusammensetzung zur Verwendung nach Anspruch 1, wobei die Zusammensetzung mehrere Isomere des Monoterpens umfasst, wobei die mehreren Isomere aus geometrischen Isomeren, Stereoisomeren, Diastereoisomeren und Kombinationen derselben ausgewählt werden.

6. Pharmazeutische Zusammensetzung zur Verwendung nach Anspruch 3, wobei das polymere Monoterpen einen Polymerisationsgrad im Bereich von mindestens 6 bis etwa 200 hat.

7. Pharmazeutische Zusammensetzung zur Verwendung nach Anspruch 6, wobei der Polymerisationsgrad mindestens etwa 25 beträgt, oder wobei der Polymerisationsgrad im Bereich von etwa 6 bis etwa 50 liegt.

8. Pharmazeutische Zusammensetzung zur Verwendung nach Anspruch 3, wobei das polymere Monoterpen ein durchschnittliches Molekulargewicht von mindestens etwa 3000 hat.

9. Pharmazeutische Zusammensetzung zur Verwendung nach Anspruch 3, wobei das polymere Monoterpen ein durchschnittliches Molekulargewicht im Bereich von mindestens etwa 1000 bis etwa 25.000 hat; wobei das durchschnittliche Molekulargewicht vorzugsweise in einem Bereich liegt, der aus der Gruppe bestehend aus von mindestens etwa 1000 bis etwa 5000; mindestens etwa 1000 bis etwa 15.000; etwa 5000 bis etwa 15.000; etwa 5000 bis etwa 20.000; etwa 15.000 bis etwa 25.000 und Kombinationen derselben ausgewählt wird.

10. Pharmazeutische Zusammensetzung zur Verwendung nach Anspruch 3, wobei das polymere Monoterpen das Produkt einer chemischen Synthese ist, die aus der Gruppe bestehend aus einer anionischen Polymerisierungsreaktion, einer kationischen Polymerisierungsreaktion, einer radikalen Polymerisierung, einer metallkatalysierten Polymerisierung und einer Fotopolymerisierungsreaktion ausgewählt wird.

11. Pharmazeutische Zusammensetzung zur Verwendung nach Anspruch 10, wobei die chemische Synthese die Verwendung eines monomeren Monoterpens als Substrat umfasst und wobei das monomere Monoterpen aus der Gruppe bestehend aus Alloocimen, Limonen, α-Pinen, β-Pinen, Geranylacetat, 3-Caren und 2-Caren ausgewählt wird.

12. Pharmazeutische Zusammensetzung zur Verwendung nach Anspruch 11, wobei das monomere Monoterpensubstrat von einer Pflanzenart abgeleitet ist.

13. Pharmazeutische Zusammensetzung zur Verwendung nach Anspruch 1, wobei die Zusammensetzung einen pharmazeutisch akzeptablen Träger umfasst, der mindestens ein Öl oder Wachs umfasst; wobei das Öl vorzugsweise ein pflanzliches Öl ist, das aus der Gruppe bestehend aus Mandelöl, Rapsöl, Kokosnussöl, Maisöl, Baumwollsamenöl, Traubenkernöl, Olivenöl, Erdnussöl, Safranöl, Sesamöl, Sojabohnenöl und Kombinationen derselben ausgewählt wird.

14. Pharmazeutische Zusammensetzung zur Verwendung nach Anspruch 1, wobei die pharmazeutische Zusammensetzung auf einem Wege verabreicht werden soll, der kein oraler oder enteraler Weg ist; oder wobei die pharmazeutische Zusammensetzung auf einem Wege verabreicht werden soll, der aus der Gruppe bestehend aus topisch, intramuskulär, intravenös, intraperitoneal, subkutan, intradermal, vaginal, rektal, intrakranial, intranasal, intraokular

und aurikular ausgewählt wird.

15. Pharmazeutische Zusammensetzung zur Verwendung nach Anspruch 1, wobei die pharmazeutische Zusammensetzung verabreicht werden soll durch Kontaktieren von Zellen einer Person mit der pharmazeutischen Zusammensetzung, und wobei die Zellen aus der Gruppe bestehend aus neuralen Zellen, neuronalen Zellen, endothelialen Zellen, epithelialen Zellen, Zellen ektodermaler Abstammung, Zellen mesodermaler Abstammung und Zellen entodermaler Abstammung ausgewählt werden; und wobei der Schritt des Kontaktierens von Zellen ex vivo oder in vitro ausgeführt wird und die Zellen danach in die Person transplantiert oder implantiert werden.

## Revendications

1. Composition pharmaceutique destinée à une utilisation thérapeutique dans le traitement d'une fonction neurologique affaiblie, dans laquelle la composition pharmaceutique comprend au moins une forme polymérique d'un monoterpène, dans laquelle le monoterpène est choisi parmi le groupe constitué de l'alloocimène, du limonène, de l'α-pinène, de l'acétate de géranyle, du 3-carène et du 2-carène, et d'un transporteur pharmaceutiquement acceptable ; et dans laquelle la composition est essentiellement dépourvue de la forme monomérique correspondante dudit monoterpène.

2. Composition pharmaceutique destinée à une utilisation selon la revendication 1, dans laquelle la fonction neurologique affaiblie est associée à un état choisi parmi le groupe constitué de la démence vasculaire, de la démence sénile, de la maladie d'Alzheimer, de la schizophrénie, de la sclérose latérale amyotrophique (ALS), de la maladie d'Huntington, de la sclérose multiple et de la maladie de Parkinson.

3. Composition pharmaceutique destinée à une utilisation selon la revendication 1, dans laquelle la composition comprend un monoterpène polymérique choisi parmi le groupe constitué de l'alloocimène polymérique, du limonène polymérique, de l'α-pinène polymérique, du β-pinène polymérique, de l'acétate de géranyle polymérique, du 3-carène polymérique, du 2-carène polymérique et de combinaisons de ceux-ci.

4. Composition pharmaceutique destinée à une utilisation selon la revendication 3, dans laquelle la composition comprend d'environ 0,01 à environ 12% (en poids) dudit monoterpène, sur la base du poids total de la composition.

5. Composition pharmaceutique destinée à une utilisation selon la revendication 1, dans laquelle la composition comprend une pluralité d'isomères dudit monoterpène, dans laquelle la pluralité d'isomères est choisie parmi des isomères géométriques, des stéréoisomères, des diastéréoisomères et des combinaisons de ceux-ci.

6. Composition pharmaceutique destinée à une utilisation selon la revendication 3, dans laquelle le monoterpène polymérique possède un degré de polymérisation compris dans la gamme allant d'au moins environ 6 à environ 200.

7. Composition pharmaceutique destinée à une utilisation selon la revendication 6, dans laquelle le degré de polymérisation est d'au moins environ 25 ; ou dans laquelle le degré de polymérisation est compris dans la gamme allant d'environ 6 à environ 50.

8. Composition pharmaceutique destinée à une utilisation selon la revendication 3, dans laquelle le monoterpène polymérique possède un nombre de poids moléculaire moyen d'au moins environ 3000.

9. Composition pharmaceutique destinée à une utilisation selon la revendication 3, dans laquelle le monoterpène polymérique possède un nombre de poids moléculaire moyen compris dans la gamme allant d'au moins environ 1000 à environ 25000 ; de préférence dans laquelle le nombre de poids moléculaire moyen compris dans la gamme choisi parmi le groupe constitué d'au moins environ 1000 à environ 5000 ; d'au moins environ 1000 à environ 15000; d'au moins environ 5000 à environ 15000 ; d'au moins environ 5000 à environ 20000 ; d'au moins environ 15000 à environ 25000 et des combinaisons de ceux-ci.

10. Composition pharmaceutique destinée à une utilisation selon la revendication 3, dans laquelle le monoterpène polymérique est le produit de la synthèse chimique choisie parmi le groupe constitué d'une réaction de polymérisation anionique, d'une réaction de polymérisation cationique, d'une polymérisation radicalaire, d'une polymérisation catalysée par un métal et d'une réaction de photopolymérisation.

**11.** Composition pharmaceutique destinée à une utilisation selon la revendication 10, dans laquelle la synthèse chimique comprend l'utilisation d'un monoterpène monomérique en tant qu'un substrat, et dans laquelle le monoterpène monomérique est choisi parmi le groupe constitué de l'alloocimène, du limonène, de l'$\alpha$-pinène, du $\beta$-pinène, de l'acétate de géranyle, du 3-carène et du 2-carène.

**12.** Composition pharmaceutique destinée à une utilisation selon la revendication 11, dans laquelle le substrat de monoterpène monomérique est dérivé d'une espèce végétale.

**13.** Composition pharmaceutique destinée à une utilisation selon la revendication 1, dans laquelle la composition comprend un transporteur pharmaceutiquement acceptable comprenant au moins une huile ou une cire ; de préférence dans laquelle l'huile est une huile végétale choisie parmi le groupe constitué de l'huile d'amande, de l'huile de canola, de l'huile de noix de coco, de l'huile de maïs, de l'huile de graine de coton, de l'huile de pépin de raisin, de l'huile d'olive, de l'huile de cacahuète, de l'huile de safran, de l'huile de sésame, de l'huile de graine de soja et de combinaisons de celles-ci.

**14.** Composition pharmaceutique destinée à une utilisation selon la revendication 1, dans laquelle la composition pharmaceutique doit être administrée par une voie qui est différente d'une voie orale ou entérale ; ou dans laquelle la composition pharmaceutique doit être administrée par une voie choisie parmi le groupe constitué de topique, intramusculaire, intraveineuse, intrapéritonéale, sous-cutanée, intradermique, vaginale, rectale, intracrânienne, intranasale, intraoculaire et auriculaire.

**15.** Composition pharmaceutique destinée à une utilisation selon la revendication 1, dans laquelle la composition pharmaceutique doit être administrée en mettant en contact des cellules d'un sujet avec la composition pharmaceutique, et dans laquelle les cellules sont choisies parme le groupe constitué de cellules neurales, de cellules neuronales, de cellules endothéliales, de cellules épithéliales, de cellules de lignée ectodermique, de cellules de lignée mésodermique et de cellules de lignée endodermique ; et dans laquelle l'étape de mise en contact de cellules est effectuée *ex vivo* ou *in vitro* et lesdites cellules sont transplantées ou implantées ci-après au sein du sujet.

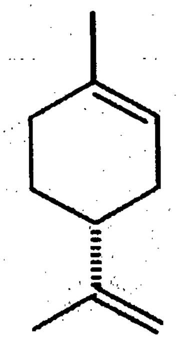

Limonene

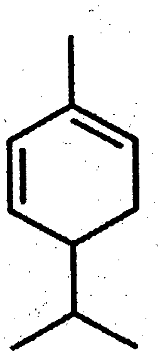

α-Phellandrene

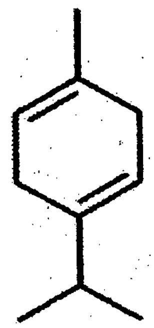

γ-Terpinene

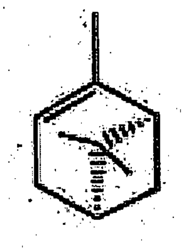

α-Pinene

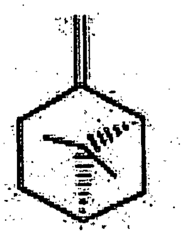

β-Pinene

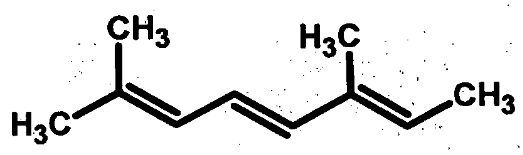

Alloocimene

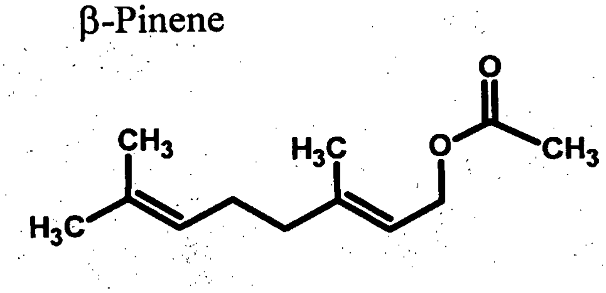

Geranyl acetate

FIGURE 1

Fig. 2A

Fig. 2B

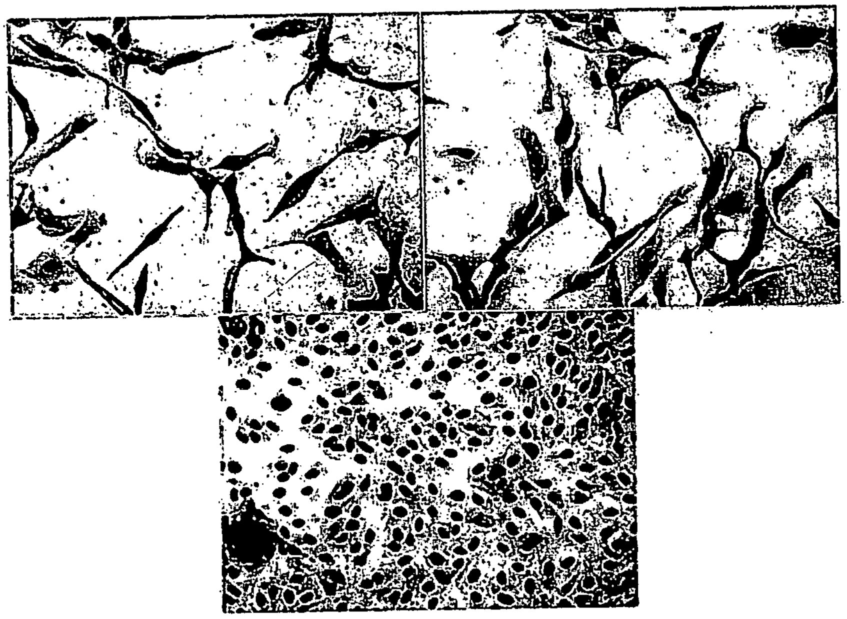

FIGURE 2

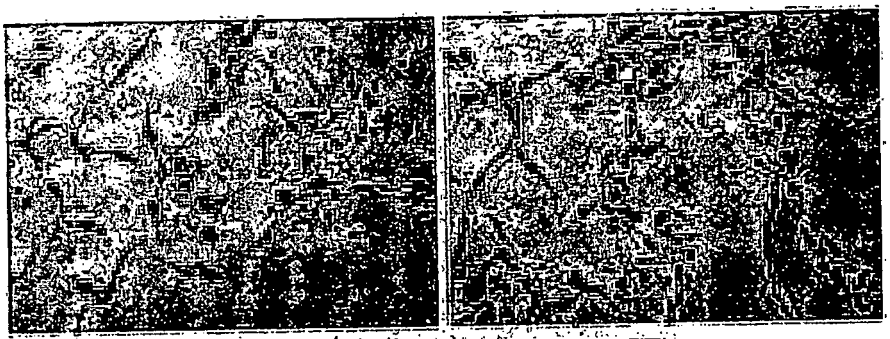

Fig. 3A

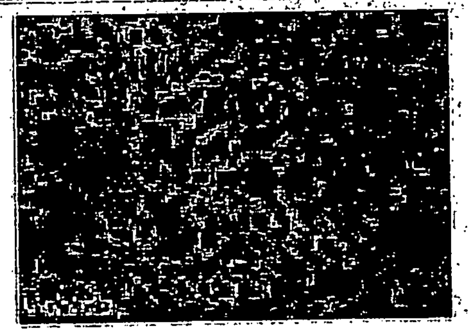

Fig. 3B

FIGURE 3

Fig. 4A

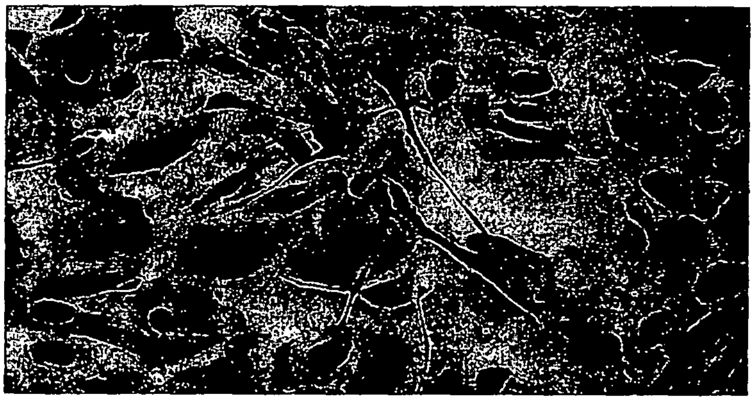

Fig. 4B

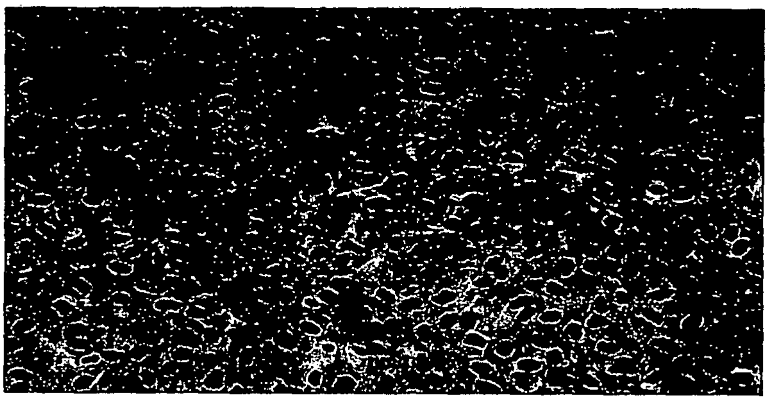

Fig. 4C

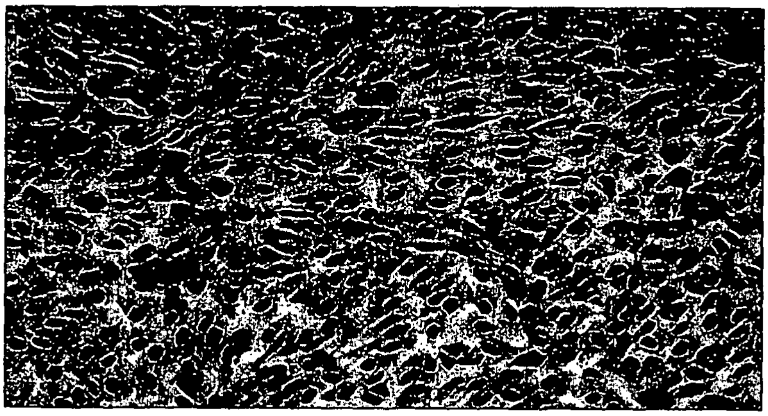

FIGURE 4

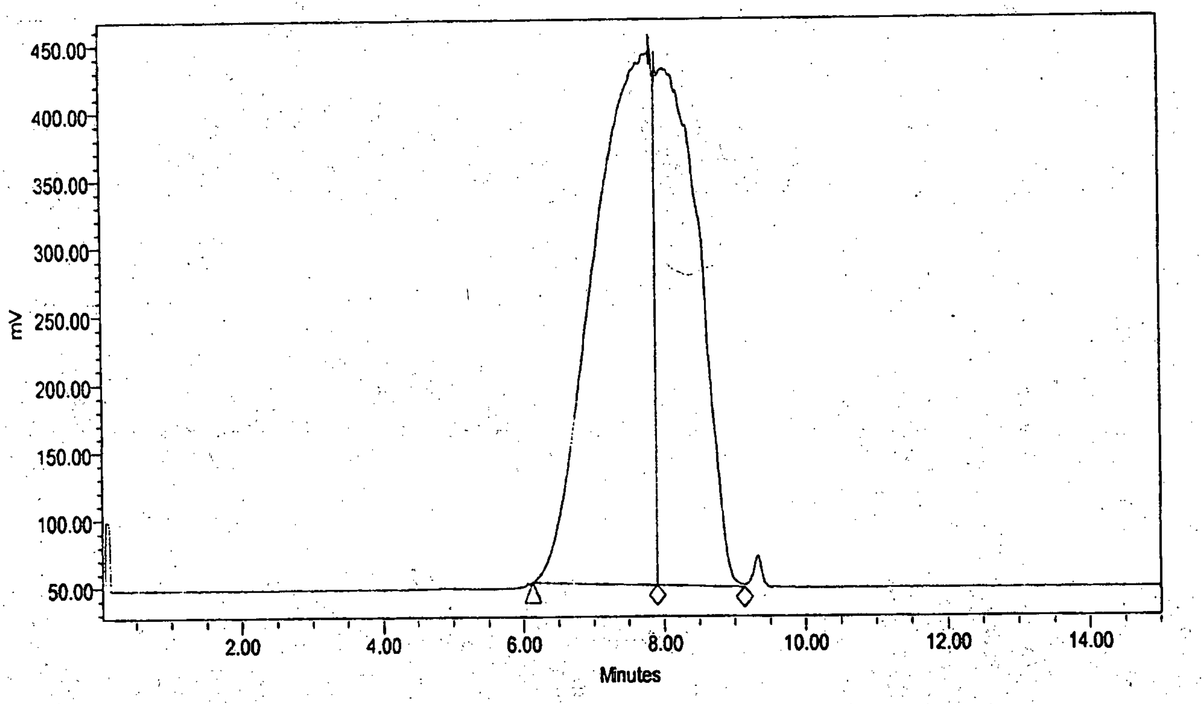
450.00
400.00
350.00
300.00
250.00
200.00
150.00
100.00
50.00
mV
2.00
4.00
6.00
8.00
10.00
12.00
14.00
Minutes

FIGURE 5

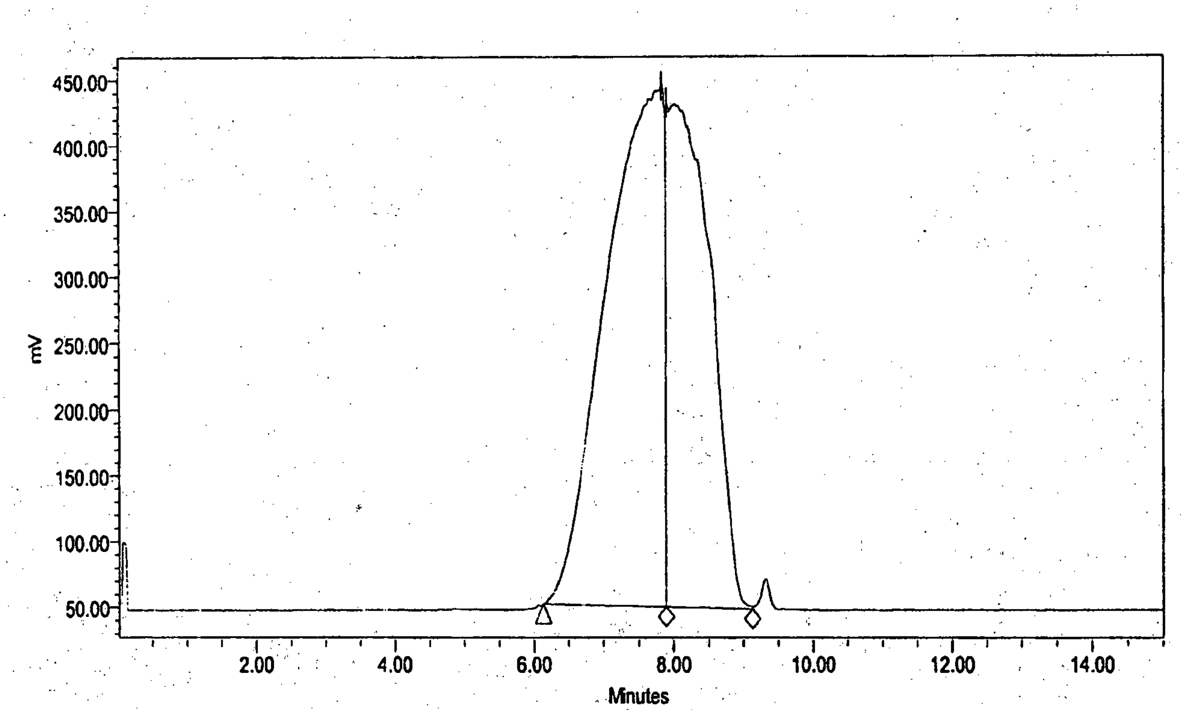
450.00
400.00
350.00
300.00
250.00
200.00
150.00
100.00
50.00
mV
2.00
4.00
6.00
8.00
10.00
12.00
14.00
Minutes

FIGURE 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7780974 B
- US 6063383 A
- US 20090304799 A
- US 20060222723 A
- US 2004052922 A
- US 20060104997 A
- WO 2004066912 A
- WO 2002051395 A
- WO 2008070783 A
- WO 1998000168 A
- US 20080121139 A
- US 5776361 A
- US 5154927 A
- US 3979371 A
- US 2264774 A
- US 4165301 A
- US 6265478 B
- US 20090209720 A
- US 4694047 A
- US 4690982 A
- US 5707648 A
- US 4713243 A
- US 5948430 A
- US 6177096 B
- US 6284264 B
- US 6592887 B
- US 6709671 B

### Non-patent literature cited in the description


- **PRAVDICH-NEMINSKAYA et al.** *Bulletin of Experimental Biology and Medicine,* January 1978, vol. 85 (1), 57-60
- **MISHRA et al.** *J Appl Polym Sci,* 2006, vol. 102, 4595-4600
- *Green Chem.,* 2006, vol. 8, 878-882
- **MILKS et al.** *J. Org. Chem.,* 1965, vol. 30 (3), 888-891
- Methods in Cell Biology
- **NEWMARK et al.** *J. Polym Sci.,* 1988, vol. 26, 71-77
- **KIMURA R ; OHNO M.** Impairments in remote memory stabilization precede hippocampal synaptic and cognitive failures in 5XFAD Alzheimer mouse model. *Neurobiol Dis.,* 05 November 2008
- **KEISARI, Y.** A colorimetric microtiter assay for the quantitation of cytokine activity on adherent cells in tissue culture. *J. Immunol. Methods,* 1992, vol. 146, 155-161